# FASCICULE DE BREVET EUROPEEN

(11) **EP 3 188 797 B1**
(45) Date de publication et mention de la délivrance du brevet: **13.12.2023**
(21) Numéro de dépôt: 15756928.6
(22) Date de dépôt: 01.09.2015
(51) Int. Cl.: A61N 7/02, A61B 5/01, A61B 5/055, A61B 18/00

(54) **SYSTEME DE STIMULATION CARDIAQUE ULTRASONORE FOCALISE**
FOKUSSIERTES ULTRASCHALL-HERZSTIMULATIONSSYSTEM
FOCUSSED ULTRASOUND CARDIAC STIMULATION SYSTEM

(30) Priorité: 02.09.2014 FR 1458210
(43) Date de publication de la demande: 12.07.2017
(73) Titulaire: Université de Bordeaux, 33000 Bordeaux (FR); Institut National de la Santé et de la Recherche Médicale (INSERM), 75013 Paris (FR); Centre Hospitalier Universitaire de Bordeaux, 33404 Talence (FR)
(72) Inventeur: MARQUET, Fabrice, F-33300 Bordeaux (FR); BOUR, Pierre, F-33400 Talence (FR); QUESSON, Bruno, F-33000 Bordeaux (FR); VAILLANT, Fanny, F-33000 Bordeaux (FR); DUBOIS, Remi, F-33700 Mérignac (FR)
(74) Mandataire: Cabinet Camus Lebkiri
(86) Numéro de dépôt international: PCT/EP2015/069964
(87) Numéro de publication internationale: WO 2016/034590

(56) Documents cités:
- EP-A2- 0 627 206
- WO-A1-2009/002492
- CN-A- 103 202 727
- US-A1- 2010 179 414

## Description

### DOMAINE

Le domaine de l'invention concerne les méthodes de calibration d'un système de génération d'un signal ultrasonore et son contrôle pour la stimulation de diverses régions du coeur. Plus particulièrement, l'invention concerne un procédé de stimulation cardiaque au moyen d'un réseau phase de signaux ultrasonores focalisés. L'invention concerne différentes applications de la stimulation par ultrasons dont notamment la resynchronisation cardiaque des ventricules ou des oreillettes, la génération d'une fibrillation ventriculaire ou auriculaire, la génération d'une extrasystole, la détection d'une zone du coeur non répondante, ou encore une application de modification du rythme cardiaque.

### ETAT DE L'ART

Aujourd'hui, il existe différentes solutions permettant de traiter les troubles du rythme cardiaque. Pour traiter ces dysfonctionnements du coeur, il est connu d'utiliser les ultrasons pour stimuler un tissu cardiaque. La stimulation peut être effectuée par la génération extracorporelle d'un signal ultrasonore au moyen d'un réseau de phase de signaux focalisés.

Le contrôle du signal ultrasonore généré est indispensable pour déterminer et traiter avec précision la région à traiter et ainsi éviter tout dommage indésirable sur les régions ciblées ou adjacentes du coeur.

A cet effet, le brevet US n° 2006/0052695 traite de la stimulation cardiaque par ultrasons contrôlée par le couplage d'un électrocardiographe mesurant l'activité électrique du coeur avec un système d'imagerie. Cette demande de brevet évoque notamment un système d'imagerie par ultrasons permettant de localiser les réponses à la stimulation en plusieurs points du coeur.

Cette solution permet d'ajuster la stimulation du coeur. L'intensité du signal ultrasonore est notamment contrôlée par la génération d'un signal ultrasonore tout d'abord de faible puissance puis par son augmentation petit à petit pour éviter toute détérioration des tissus.

Un inconvénient de cette méthode est qu'elle ne permet pas de quantifier les dommages thermiques et mécaniques causés par la stimulation.

Par ailleurs un autre problème des solutions connues de l'art antérieur est qu'elles ne permettent pas de générer des configurations de signaux ultrasonores pouvant créer une stimulation cardiaque sans générer de dommage thermique dans une zone ciblée. WO 2009/002492, EP0627206, CN103202727, et US2010/179414 divulguent l'art antérieur.

### RESUME DE L'INVENTION

L'invention est définie par la revendication indépendante 1. Les méthodes mentionnées ci-après ne font pas partie de l'invention revendiquée.

L'invention vise à pallier les inconvénients précités. Notamment, l'invention permet de mesurer ou d'anticiper les dommages mécaniques et thermiques des tissus cardiaques après l'exposition d'une zone du coeur à un réseau de phase de signaux ultrasonores focalisés ou à un générateur de signaux ultrasonores. Une particularité d'un mode de réalisation de l'invention est de contrôler la température et/ou ses variations dans une zone sensiblement confondue avec ou proche de la zone focalisée pour prévenir d'un dommage des tissus.

Un objet de l'invention concerne une méthode de contrôle d'une zone du coeur. La méthode comprend :
▪ une acquisition du rythme de l'électrocardiogramme du coeur ;
▪ une acquisition d'au moins une image d'une région du coeur dans laquelle une zone ciblée est repérée, ladite au moins une image étant acquise de manière synchrone avec le rythme de l'électrocardiogramme du coeur par un système d'imagerie ;
▪ une génération d'un premier faisceau de signaux ultrasonores focalisé dans la zone ciblée lesdits faisceaux étant émis par un réseau de phase, lesdits signaux étant configurés en phase pour générer au moins une impulsion dans une zone focalisée, ladite au moins une impulsion étant synchronisée sur le rythme de l'électrocardiogramme (ECG) du coeur, l'impulsion ayant une amplitude et une durée prédéfinies ;
▪ une détermination d'une température de la zone focalisée à partir d'une acquisition d'au moins une image au moyen d'un système d'imagerie ;
▪ une détermination d'une déformation tissulaire dans la zone focalisée en réponse à au moins une impulsion du premier faisceau focalisé.

Selon un autre mode de réalisation, l'invention comprend une étape de détermination d'un niveau de cavitation dans la zone focalisée en réponse à au moins une impulsion du premier faisceau focalisé. Cette étape peut être réalisée alternativement à l'étape de détermination d'une déformation tissulaire précédemment indiquée. En outre, cette étape peut être également conduite conjointement à la détermination d'une déformation tissulaire. Dans ce dernier cas, les deux mesures peuvent être corroborées pour qualifier la zone stimulée.

Un avantage d'une telle méthode est d'effectuer un contrôle de certains paramètres tels que la température, la déformation tissulaire et/ou l'activité électrique et/ou le niveau de cavitation sur la zone ciblée afin de calibrer le faisceau de signaux ultrasonores en générant l'amplitude et la durée adéquates lors de la stimulation cardiaque.

Un avantage d'un mode de réalisation du procédé de l'invention est l'utilisation d'un dispositif d'imagerie par IRM permettant d'obtenir une information de température et une information de déplacement des tissus liées à la poussée ultrasonore. Le procédé de l'invention permet donc à partir d'un même équipement d'effectuer un contrôle visuel des dommages éventuels et d'asservir le réseau de phase de signaux focalisés.

Selon un autre mode de réalisation, un système d'imagerie tel qu'un système échographique permet d'obtenir une information de température au niveau de la zone focalisée et dans les zones voisines de la zone focalisée.

Selon un mode de réalisation, la méthode de contrôle comprend :
▪ une détermination d'une activité électrique dans la zone focalisée en réponse à au moins une impulsion, ladite au moins impulsion ayant une durée inférieure ou égale à 1 ms.

Selon un mode de réalisation, la méthode de contrôle comprend :
▪ un asservissement dynamique de la position de la zone focalisée sur la position de la zone ciblée au moyen d'un système de positionnement permettant de mesurer des mouvements respiratoires du coeur dans un référentiel lié au réseau de phase et d'en déduire un paramètre de compensation pour calculer une nouvelle position de la zone ciblée, ledit réseau de phase appliquant automatiquement un paramètre de phase à chaque signal pour défléchir le faisceau vers la nouvelle position de la zone ciblée.

Selon un mode de réalisation, la méthode de contrôle comprend :
▪ une détermination par un système d'imagerie de l'image de la paroi transcostale projetée dans un plan image du réseau de phase en prenant en considération la position et l'orientation du réseau de phase ;
▪ une activation ou une désactivation de chaque élément du réseau de phase en fonction de la position desdits éléments vis-à-vis de la position de l'image projetée de la paroi transcostale.

Selon un mode de réalisation, la méthode de contrôle comprend :
▪ Un asservissement dynamique de la désactivation et de l'activation des éléments du réseau de phase en fonction du calcul de chaque paramètre de phase appliqué à chacun des signaux.

Selon un mode de réalisation, la température dans la zone focalisée et la déformation tissulaire sont déterminées par un même système d'imagerie, ledit système d'imagerie étant un système d'imagerie IRM, les données acquises par le système d'imagerie IRM permettant de déduire une déformation locale du tissu induite par la pression ultrasonore générée par le faisceau de signaux ultrasons et une élévation de température locale induite par l'énergie générée localement par le faisceau de signaux ultrasons.

Selon un mode de réalisation, une comparaison de la position de la zone focalisée déterminée par un système d'imagerie et de la position de la zone ciblée déterminée par le système de positionnement génère au moins une donnée pour calibrer des éléments du réseau de phase de manière à faire correspondre une position de la zone focalisée avec la position de la zone ciblée.

Selon un mode de réalisation, le système de positionnement peut être notamment :
▪ soit un système d'imagerie IRM, les positions étant calculées à partir d'un traitement d'images ;
▪ soit un système de positionnement comprenant au moins un émetteur émettant des ondes ultrasonores et une pluralité de capteurs à ultrasons détectant les ondes réfléchies, les positions étant déterminées par triangulation.

Ce système de positionnement est avantageusement utilisé pour le procédé de contrôle et le procédé de stimulation.

Selon un mode de réalisation, la méthode de contrôle comprend une mesure de l'activité électrique générée par la dépolarisation électrique induite par l'application d'au moins une impulsion ultrasonore dans la zone ciblée.

Selon un mode de réalisation, la méthode de contrôle comprend une calibration d'un signal généré dans la zone focalisée par la définition de paramètres comprenant au moins un niveau de l'amplitude et une durée d'une impulsion en fonction d'au moins une donnée parmi lesquelles :
▪ une consigne de température dans la zone focalisée et/ou sur les zones voisines et/ou sur les côtes de la paroi transcostale ; et/ou
▪ une consigne de la déformation tissulaire dans la zone focalisée et/ou ;
▪ une consigne d'un niveau de cavitation dans la zone focalisée et/ou;
▪ une détection d'un mouvement de la zone focalisée dans un repère lié au réseau de phase et/ou ;
▪ une consigne d'activité électrique dans la zone focalisée.

Selon un mode de réalisation, l'activité électrique générée par le faisceau focalisé (F_{US}) et mesurée dans ou à proximité de la zone ciblée est corrélée avec les mesures de déformations tissulaires dans la même zone ciblée obtenues par le système d'imagerie, ladite corrélation permettant de déterminer un indicateur d'activité mécano-électrique du tissu cardiaque d'une zone ciblée donnée.

Selon un mode de réalisation, la génération d'un premier faisceau de signaux ultrasonores focalisés dans la zone ciblée est engagée de sorte que la au moins une impulsion soit générée lors de la repolarisation des tissus cardiaques dans la zone ciblée.

Selon un mode de réalisation, la méthode comprend une calibration d'un premier signal focalisé comprenant une pluralité d'impulsions, chaque impulsion ayant une première durée, une amplitude, ledit signal étant appliqué pendant une durée dans une zone ciblée.

Selon un mode de réalisation, la méthode de contrôle est effectuée dans différentes zones ciblées du coeur, la méthode comprenant en outre après l'application d'un faisceau de signaux focalisés:
▪ Un relevé de différentes valeurs représentant soit des déformations tissulaires de chaque zone ciblée, soit des niveaux électriques mesurés à proximité ou dans chaque zone ciblée
▪ Un relevé des temps de réponse électrique ou de déformations de chaque zone ciblée ;
▪ Une calibration pour chaque zone ciblée de signaux, lesdits signaux étant configurés entre eux avec un délai temporel dépendant des relevés des temps de réponse.

Selon un mode de réalisation, au moins une zone ciblée du coeur est indiquée comme « non répondante » lorsque la déformation tissulaire ou l'activité électrique ou le niveau de cavitation dans cette zone est inférieure à un seuil prédéterminé pour une amplitude et une durée donnée d'au moins une impulsion du faisceau focalisé et une température donnée dans la zone focalisée.

Le procédé de l'invention concerne également une méthode de stimulation cardiaque, également appelée méthode de stimulation électrique. La méthode de stimulation électrique peut comprendre avantageusement les caractéristiques définissant les modes de réalisation de la méthode de contrôle. En particulier, les équipements utilisés pour les deux méthodes peuvent être identiques et les paramètres mesurés et les configurations peuvent être similaires.

La méthode de stimulation cardiaque d'une zone donnée d'un coeur par génération d'un faisceau de signaux ultrasonores focalisé, comprend :
▪ une acquisition du rythme de l'électrocardiogramme du coeur ;
▪ une détermination d'au moins une position d'une zone ciblée dans le coeur ;
▪ une génération d'un faisceau de signaux ultrasonores focalisé et synchrone avec le rythme de l'électrocardiogramme dont :
   ▪ une amplitude des impulsions est configurée de sorte que la pression acoustique appliquée dans la zone focalisée est comprise dans une première gamme de pressions de [2 - 12 MPa] ;
   ▪ une durée des impulsions est comprise dans une première gamme de durées de [50 µs - 50 ms];
   ▪ la durée d'application du faisceau focalisé étant supérieure à 50 µs ;
▪ un asservissement de la position de la zone focale du faisceau sur la position calculée en temps réel de la zone ciblée au moyen d'un système de positionnement ;
▪ un contrôle actif en temps réel :
   ▪ d'une température dans la zone focalisée à partir d'une acquisition d'image(s) ne dépassant pas un seuil prédéterminé ;
   ▪ d'une déformation tissulaire après chaque impulsion comprise dans une gamme de valeurs prédéfinie à partir d'un système d'imagerie et/ou d'une activité électrique et/ou d'un niveau de cavitation dans la zone focalisée mesurée après chaque impulsion comprise dans une gamme de valeurs prédéfinie ;
   ▪ d'un paramètre de synchronisation assurant le respect de la synchronisation du rythme de l'électrocardiogramme sur le rythme des impulsions du faisceau focalisé.

En particulier le procédé de l'invention comprend deux modes de génération de signaux qui sont regroupés selon des gammes de valeurs et pour lesquels les dommages thermiques et mécaniques peuvent être contrôlés en temps réel ou anticipés par une pré-configuration.

Selon un mode de réalisation, la température dans la zone focalisée Z_{F} et la déformation tissulaire sont déterminées par un même système d'imagerie, ledit système d'imagerie étant, par exemple, un système d'imagerie IRM.

L'amplitude des impulsions est configurée de sorte que la pression acoustique appliquée dans la zone focalisée est comprise dans une première gamme de pressions de [2 - 8 MPa] pour des durées d'impulsions comprises dans une première gamme de durées [1ms - 50 ms], les dites impulsions générées provoquant une stimulation électrique tout en maintenant un seuil de déformation mécanique du tissu cardiaque inférieure à une valeur seuil, ladite valeur seuil étant calculée à partir d'un paramètre correspondant à une proportion donnée d'une contraction ou d'une relaxation ou d'une élasticité du tissu.

L'amplitude des impulsions est configurée de sorte que la pression acoustique appliquée dans la zone focalisée est comprise dans une seconde gamme de pressions de [6 - 12 MPa] pour des durées d'impulsions comprises dans une seconde gamme de durées [50µs - 1ms], lesdites impulsions générées provoquant une stimulation électrique dans les tissus cardiaques tout en maintenant localement dans la zone ciblée un seuil de température inférieur à une température seuil.

Selon un mode de réalisation, un second contrôle de zones voisines à la zone focalisée est réalisé, ledit second contrôle comportant la mesure d'au moins un des paramètres suivants en temps réel :
▪ une température d'au moins une zone voisine de la zone focalisée et/ou ;
▪ une déformation tissulaire dans au moins une zone voisine de la zone focalisée en réponse à au moins une impulsion du premier faisceau focalisé et/ou ;
▪ une activité électrique dans au moins une zone voisine de la zone focalisée en réponse à au moins une impulsion du premier faisceau focalisé et/ou ;
▪ un niveau de cavitation dans au moins une zone voisine de la zone focalisée en réponse à au moins une impulsion du premier faisceau focalisé.

Selon un mode de réalisation, la méthode de stimulation de l'invention comprend préalablement :
▪ une calibration d'un faisceau focalisé selon le procédé de contrôle de l'invention ;
▪ ladite calibration permettant de déterminer les paramètres suivants :
   ▪ une amplitude d'impulsions configurée de sorte que la pression acoustique appliquée dans la zone focalisée est comprise dans la première gamme de pressions de [2 - 12 MPa] ;
   ▪ une durée d'application du faisceau focalisé sur une période supérieure à 50 µs.

Selon un exemple, la durée des impulsions est comprise dans une première gamme de durées G_{D1} de [50 µs - 50 ms].

Selon un mode de réalisation, la méthode de stimulation comprend :
▪ une détermination de la position dans l'espace d'une ou de plusieurs zone(s) ciblée(s) au moyen d'une analyse d'une pluralité d'images acquises par un système d'imagerie IRM ;
▪ un asservissement des faisceaux focalisés au cours du temps sur la ou les zone(s) ciblée(s).

Selon un mode de réalisation, la méthode de stimulation comprend :
▪ une détermination de la position dans l'espace de la zone ciblée au moyen d'une analyse des signaux par des sondes ultrasonores ;
▪ un asservissement des faisceaux focalisés au cours du temps sur la zone ciblée.

Selon un mode de réalisation, la méthode de stimulation comprend :
▪ une détermination de la position dans l'espace de la zone ciblée (Z_{c}) au moyen d'une analyse des signaux par des cathéters intracardiaques introduits dans le coeur dont une sonde est positionnée à proximité de la zone ciblée (Z_{c});
▪ un asservissement des faisceaux focalisés (F_{US}) au cours du temps sur la zone ciblée (Z_{c}).

Selon un mode de réalisation, la méthode de stimulation est appliquée pour la resynchronisation des oreillettes ou la resynchronisation des ventricules. A cet effet, la méthode de stimulation comprend :
▪ une acquisition d'un électrocardiogramme du coeur indiquant le rythme de l'électrocardiogramme du coeur ;
▪ une détermination de une ou plusieurs zones ciblées dans le(s)quelle(s) le faisceau de signaux ultrasonore calibré est généré ;
▪ une synchronisation de chaque impulsion du faisceau focalisé sur le rythme de l'électrocardiogramme du coeur, chaque impulsion étant générée entre la fin de la repolarisation et le début de la dépolarisation électriques d'un ventricule ou d'une oreillette.

Selon un mode de réalisation, la méthode de stimulation est appliquée à la génération d'une fibrillation cardiaque. A cet effet, la méthode de stimulation comprend :
▪ une acquisition d'un électrocardiogramme d'un ventricule du coeur indiquant le rythme de l'électrocardiogramme du coeur ;
▪ une détermination d'au moins une zone ciblée dans laquelle le faisceau de signaux ultrasonore calibré est généré ;
▪ une synchronisation de chaque impulsion du faisceau focalisé sur une période pendant laquelle les tissus cardiaques se polarisent ;
▪ une analyse de la réponse électrique de la zone ciblée et de l'ECG de manière à vérifier la génération d'une fibrillation ventriculaire ou auriculaire.

Selon un mode de réalisation, la méthode de stimulation est appliquée à la détection d'une zone génératrice d'une extrasystole. A cet effet, la méthode de stimulation comprend :
▪ une acquisition d'un électrocardiogramme d'un coeur indiquant le rythme de l'électrocardiogramme du coeur, ledit électrocardiogramme comportant la présence d'une extrasystole se produisant à un instant identifié de l'électrocardiogramme;
▪ une détermination d'au moins une zone ciblée dans laquelle le faisceau de signaux ultrasonore calibré est généré ;
▪ une synchronisation de chaque impulsion du faisceau focalisé sur l'instant identifié de l'électrocardiogramme du coeur;
▪ une analyse de la réponse électrique ou de la déformation tissulaire de manière à identifier si la zone ciblée est génératrice de l'extrasystole.

Selon un mode de réalisation, la méthode de stimulation est appliquée à la modification de la fréquence cardiaque. A cet effet, la méthode de stimulation comprend :
▪ une acquisition d'un électrocardiogramme d'un coeur indiquant le rythme de l'électrocardiogramme du coeur ;
▪ une détermination d'une pluralité de zones ciblées dans lesquelles le faisceau de signaux ultrasonores calibré est généré ;
▪ une génération d'un faisceau de signaux focalisé dont la fréquence d'impulsions est différente de la fréquence de l'électrocardiogramme de manière à synchroniser le rythme cardiaque sur les impulsions du faisceau focalisé.

Un autre objet de l'invention concerne un système de stimulation cardiaque ultrasonore comprenant :
▪ un moyen de mesure de l'activité électrique du coeur pour l'acquisition d'un électrocardiogramme ;
▪ un moyen de génération d'un faisceau de signaux ultrasonores focalisé sur une zone ciblée, lesdits signaux étant calibrés pour générer une stimulation électrique dans une zone du coeur, ladite génération du faisceau étant synchrone avec un premier instant choisi de l'électrocardiogramme ;
▪ un moyen de localisation de la zone ciblée couplé à un moyen de positionnement du moyen de génération du faisceau focalisé de sorte à asservir ledit faisceau de signaux ultrasonores focalisé dans la zone ciblée, ledit moyen de localisation étant synchronisé avec le moyen de génération du faisceau de signaux focalisés ;
▪ un même moyen de contrôle capable de suivre en temps réel une température et une déformation tissulaire dans la zone ciblée, ledit moyen de contrôle relevant des mesures de manière synchrone avec le rythme de l'électrocardiogramme.

Selon un mode de réalisation, le système de positionnement et le moyen de contrôle de la température sont un même système d'imagerie IRM.

Le système de l'invention permet de mettre en oeuvre les étapes du procédé de contrôle et du procédé de stimulation de l'invention.

Enfin, l'invention concerne un dispositif portable de stimulation cardiaque répondant à des situations d'urgence. Le dispositif portable peut avantageusement être pré-calibré selon le procédé de contrôle de l'invention.

Le dispositif portable de stimulation cardiaque par ultrasons est destiné à être apposé au contact de la peau d'un patient. Il comprend un système électrique permettant une mesure d'une activité cardiaque indiquant au moins la fréquence de battement du coeur le cas échéant, un générateur de signaux ultrasons, un capteur d'effet mécanique permettant de mesurer la présence d'une contraction du coeur, ledit dispositif portable comprenant au moins une pré-configuration définissant au moins un train d'impulsions de signaux ultrasonores synchronisés sur la fréquence cardiaque acquise par le système électrique, ledit dispositif comprenant un moyen d'activation du générateur de signaux ultrasonores.

### BREVE DESCRIPTION DES FIGURES

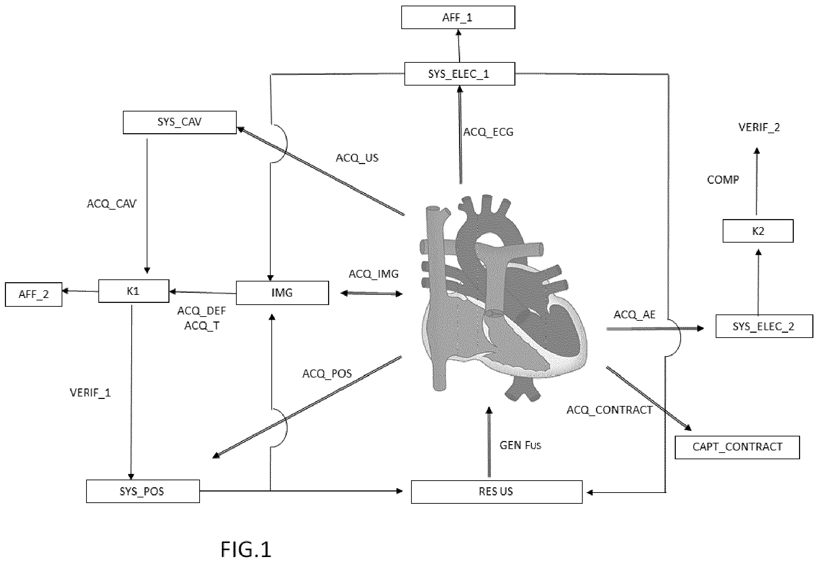

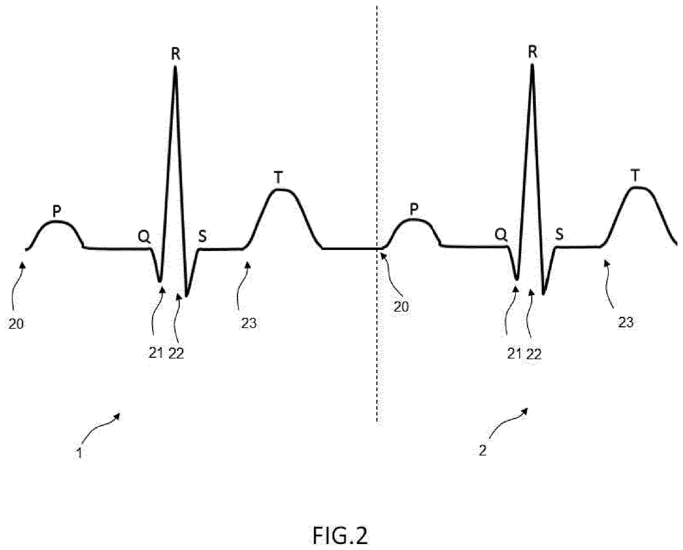

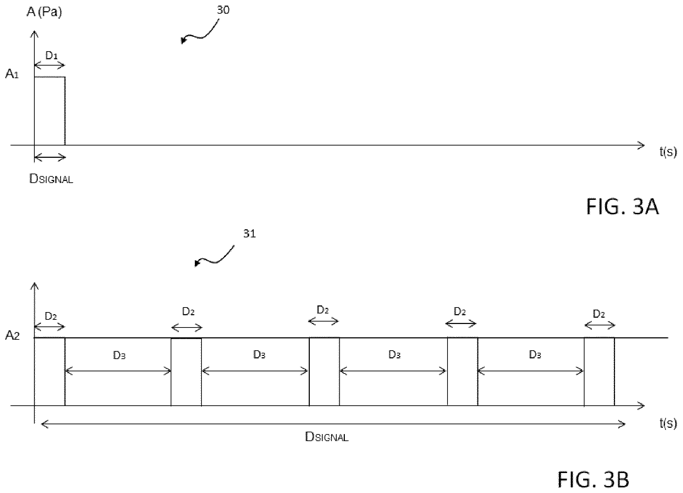

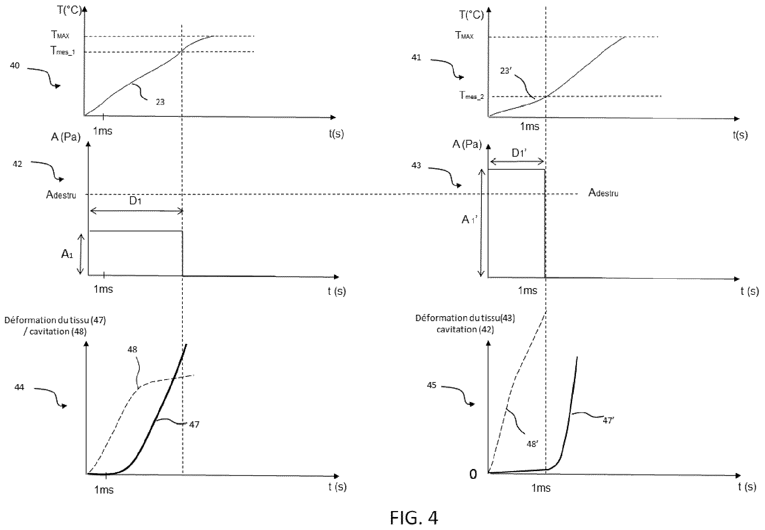

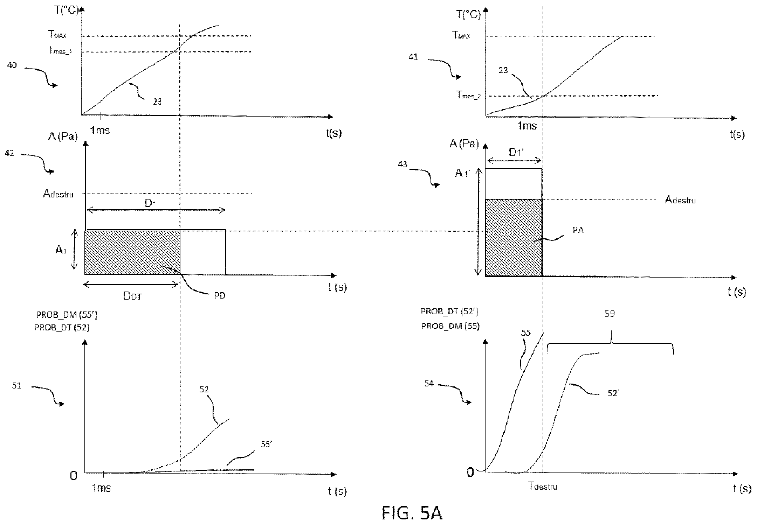

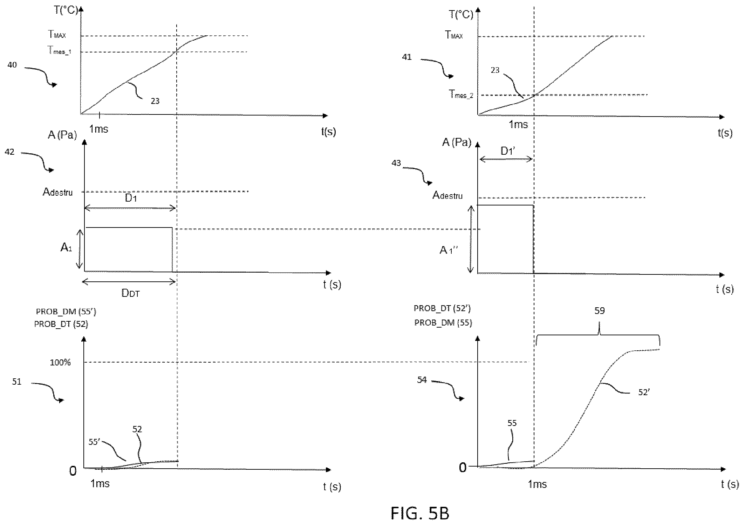

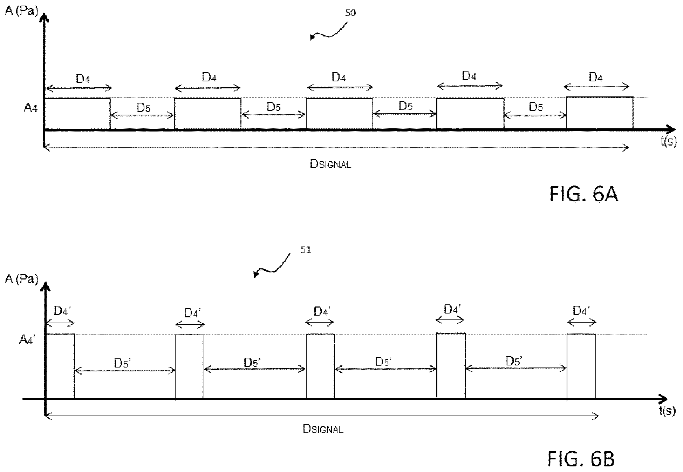

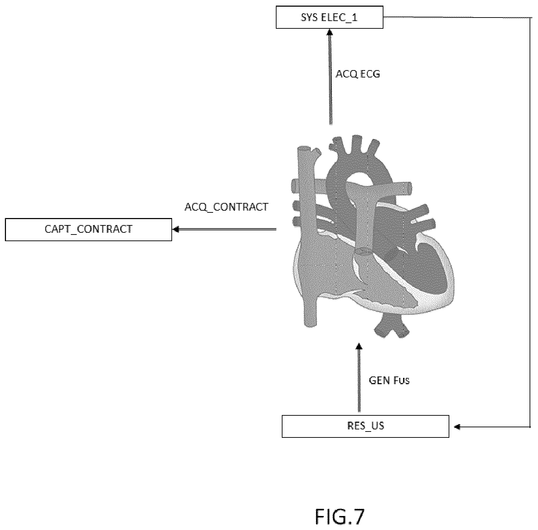

D'autres caractéristiques et avantages de l'invention ressortiront à la lecture de la description détaillée qui suit, en référence aux figures annexées, qui illustrent :
▪ figure 1: un schéma général de fonctionnement du procédé de l'invention selon un mode de réalisation ;
▪ figure 2 : un exemple d'une séquence d'un électrocardiogramme du coeur ;
▪ figure 3A : un diagramme représentant l'amplitude et la durée d'une impulsion d'un faisceau de signaux ultrasonores appliquée sur une zone ciblée du coeur pour le contrôle de ladite zone;
▪ figure 3B : un diagramme représentant l'amplitude et la durée d'une pluralité d'impulsions d'un faisceau de signaux ultrasonores appliquées sur une zone ciblée du coeur pour la stimulation de ladite zone ;
▪ figure 4 : les diagrammes représentant l'évolution de la température, de la déformation du tissu au niveau de la zone ciblée du coeur selon l'amplitude et la durée des impulsions d'un faisceau de signaux ultrasonores sur ladite zone ;
▪ figure 5A, 5B : les diagrammes représentant l'évolution de la température, des dommages mécaniques et/ou thermiques au niveau de la zone ciblée selon l'amplitude et la durée des impulsions d'un faisceau de signaux ultrasonores sur ladite zone ;
▪ figure 6A : un diagramme représentant la stimulation d'une zone ciblée du coeur pour laquelle les impulsions sont de faibles amplitudes et de longues durées ;
▪ figure 6B : un diagramme représentant la stimulation d'une zone ciblée du coeur pour laquelle les impulsions sont de fortes amplitudes et dont les durées d'impulsion sont courtes ;
▪ figure 7 : un dispositif portable de stimulation cardiaque par ultrasons.

### DESCRIPTION

### Définitions et introduction du principe

La figure 1 représente un schéma général de fonctionnement d'un mode de réalisation du procédé de l'invention. Le système de la figure 1 représente différents équipements qui sont décrits successivement.

On nommera dans la suite de la description indifféremment une « amplitude » et une « pression acoustique ».

Une « impulsion » peut s'interpréter au niveau du signal comme une émission pendant une certaine durée mais aussi comme le phénomène induit localement par ce signal. Le phénomène induit correspond à une poussée ultrasonore locale résultant d'une force de radiation ultrasonore. Cette dernière poussée est susceptible d'exercer directement une poussée mécanique sur les tissus cardiaques.

Un réseau de phase RES_US permet de générer un ou plusieurs faisceaux focalisés dans une ou plusieurs zones focalisées. Chaque zone focalisée est asservie en position, par exemple, au moyen d'un système de positionnement SYS_POS générant une consigne dynamiquement au réseau de phase.

On appelle « faisceau focalisé » : un ensemble de signaux provenant de différents éléments d'un réseau de phase dont les caractéristiques en fréquence et en phase permettent de générer des interférences constructives ou cohérentes en une zone focalisée.

Le réseau de phase permet, selon les modes de réalisations, de définir une ou plusieurs zones focalisées dans le coeur grâce à une configuration particulière des phases de chaque élément du réseau de phase. Les différentes zones focalisées sont obtenues en faisant interférer les signaux de différents éléments du réseau de phase en différents points.

Les procédés de l'invention permettent d'effectuer des mesures de paramètres de contrôle, tels que la déformation tissulaire ACQ_DEF, la température ACQ_T, l'activité électrique ACQ_AE ou encore la présence d'une contraction cardiaque ACQ_CONTRACT, pendant ou conséquemment à l'application du faisceau focalisé. En outre, les procédés de l'invention permettent de mesurer un niveau représentatif du phénomène de cavitation ACQ_CAV dans ou à proximité de la zone focalisée ainsi qu'une mesure de position ou de déplacement de la zone ciblée ACQ_POS.

On parle indifféremment de « déformation tissulaire » et de « déplacement des tissus » dans la description pour décrire l'effet causé par la génération d'une poussée ultrasonore sur les tissus cardiaques.

L'asservissement de la ou des position(s) du ou des faisceaux focalisés en un ou plusieurs point(s), appelé(s) « zone focalisée », est réalisée au moyen d'une consigne de position, appelée « zone ciblée ».

Le mécanisme d'asservissement du faisceau focalisé sur une zone ciblée est avantageusement réalisé de manière synchrone entre le système de positionnement et le réseau de faisceaux grâce à l'acquisition de l'ECG qui constitue une référence temporelle commune aux différents équipements.

Le procédé de l'invention permet de prendre en compte un paramètre de déplacement de la consigne de position de la zone ciblée Z_{C} causé par les mouvements respiratoires et/ou la contraction du coeur. Une nouvelle position ciblée est alors calculée au moyen d'un calculateur couplé au moyen de positionnement pour adapter la consigne au réseau de phase. Selon le procédé de l'invention, le réseau de phase est capable de générer un paramètre de phase à chaque signal émis par un élément du réseau pour défléchir le faisceau et asservir le point focalisé Z_{F} sur la nouvelle position de la zone ciblée Z_{C}.

Par ailleurs, outre les phénomènes respiratoires, il subsiste des mouvements de contractions du coeur, particulièrement pendant le complexe QRS de l'ECG. De manière à s'affranchir des mouvements de contraction du coeur dans l'asservissement de la zone focalisée sur la zone ciblée, le faisceau focalisé peut :
▪ soit être interrompu lors de l'apparition du complexe QRS visible sur l'ECG ;
▪ soit être complété par l'application d'une seconde correction de déflexion pour compenser le déplacement du coeur lors de l'apparition du complexe QRS.

L'invention permet de mettre en oeuvre les deux solutions lors du procédé de contrôle ou lors du procédé de stimulation.

Selon un mode de réalisation, l'émetteur peut comprendre un unique émetteur transmettant un signal dans la zone focalisée.

### Acquisition ECG

Une première étape, notée ACQ_ECG, comprend l'acquisition d'un électrocardiogramme, noté ECG, d'un patient ou d'un animal à partir d'un premier système électrique noté SYS_ELEC_1. Selon un mode de réalisation, l'électrocardiogramme permet d'acquérir le rythme des battements d'un coeur, appelé également la fréquence cardiaque, qui s'affiche sur un afficheur, noté AFF_1.

Selon un mode de réalisation, l'acquisition de l'ECG est réalisée en plaçant des électrodes à la surface du corps d'un patient ou d'un animal. Cette solution permet de mesurer l'activité électrique d'un coeur de manière non invasive. Selon une variante de réalisation, l'ECG peut être acquis au moyen de douze ou seize dérivations selon des modalités connues d'agencement et de positionnement d'électrodes.

Selon un mode de réalisation, un cathéter spécifique est positionné dans une cavité du coeur pour la mesure d'une activité électrique locale. Dans ce cas, le procédé de l'invention permet également de comparer les activités électriques acquises par le cathéter électrique et le dispositif permettant d'obtenir l'ECG. Ce moyen est noté SYS_ELEC_2 sur la figure 1.

### - Synchro applications

Outre son affichage, l'ECG est utilisé selon le procédé de l'invention pour synchroniser différents équipements ensemble. La synchronisation des différents équipements permet de générer des actions de manière synchrone dans une ou plusieurs régions du coeur.

Les actions synchronisées comprennent notamment :
▪ la balistique : c'est-à-dire l'asservissement de la position de la zone focalisée Z_{F} sur la position de la zone ciblée Z_{C} ou en prenant en compte par exemple la fréquence de contraction du coeur dans l'asservissement de la balistique ou l'arrêt de l'émission des faisceaux. Cette synchronisation prend en compte les mouvements du coeur ainsi qu'au besoin l'évitement de la paroi transcostale et/ou ;
▪ le contrôle : c'est-à-dire la mesure de certains paramètres de contrôle pendant ou conséquemment à la génération du faisceau.

### - Synchronisation du faisceau

Dans un mode de réalisation, la génération GEN F_{US} d'un faisceau de signaux ultrasonores focalisé dans une zone ciblée du coeur et l'acquisition d'images ACQ IMG sont synchronisées avec l'ECG. On entend par « synchronisé » du point de vue du réseau de phase, le fait que la génération du faisceau ultrasonore GEN F_{US} est déclenchée à un instant déterminé de l'ECG. Cette synchronisation permet de provoquer un effet physiologique souhaité dans une zone du coeur en prenant en considération l'état de polarisation du tissu cardiaque. Lorsque des impulsions de faisceaux ultrasonores F_{US} sont générées périodiquement, la synchronisation des impulsions sur l'ECG permet de provoquer une stimulation dans un même état de polarisation des tissus cardiaques à chaque émission.

La synchronisation de l'ECG et du réseau de phase est donc configurée pour obtenir un effet souhaité du faisceau sur une zone du coeur selon l'application envisagée. C'est-à-dire que la génération du faisceau focalisé est générée dans une fenêtre de temps correspondant à une dépolarisation ou une repolarisation d'un ventricule ou d'une oreillette selon le cas de figure.

### - Synchronisation de l'imagerie

En ce qui concerne l'acquisition d'au moins une image ACQ IM, elle est préférentiellement synchronisée avec l'ECG également. C'est-à-dire que l'image est acquise à des instants donnés de l'ECG. Les instants d'acquisition d'image vis-à-vis de l'ECG peuvent être configurés par un opérateur ou peuvent être automatiquement déduits en fonction d'une consigne de synchronisation.

### Acquisition d'images

Une seconde étape, notée ACQ IMG, comprend l'acquisition d'au moins une image d'une région du coeur, par un système d'imagerie noté IMG, dans laquelle une zone ciblée Z_{C} est repérée, notée ACQ_Zc.

Selon un mode de réalisation, l'acquisition de l'image est réalisée au moyen d'un système d'imagerie IRM. Basé sur les propriétés magnétiques des atomes, le système d'imagerie IRM consiste à appliquer un champ magnétique aux noyaux atomiques puis à stimuler par radiofréquences lesdits noyaux atomiques. La reconstitution d'une image est alors possible à partir du signal émis lors de phase de relaxation et recueilli par des capteurs électromagnétiques.

Avantageusement, le système d'imagerie IRM couplé avec un calculateur K₁ permet de :
▪ définir une position d'une zone ciblée Z_{C} comme une consigne d'asservissement du réseau de phase de signaux focalisés ; la position de la zone ciblée Zc peut également être définie par un autre équipement de positionnement ou être transmise à un tel équipement ;
▪ déduire une déformation tissulaire et/ou une variation de déformation tissulaire à proximité ou dans la zone focalisée, l'IRM dans ce mode est appelée plus communément IRM-ARFI ; l'IRM ARFI permet de mesurer la dureté ou l'élasticité des tissus suite à une augmentation de la température dans une zone identifiée ;
▪ déduire une température et/ou une variation de températures à proximité ou dans la zone focalisée, l'IRM dans ce mode est appelée plus communément IRM-T ;
▪ déterminer la projection de la paroi transcostale d'un patient dans le plan image du réseau de phase pour activer ou désactiver les éléments du réseau de phase susceptibles de causer des dommages à la paroi osseuse en prenant en considération un paramètre de déflexion du faisceau.

Selon un autre mode de réalisation, l'invention comprend une acquisition d'une image au moyen d'un système d'imagerie ultrasonore. Ce système peut être utilisé en complément d'un système d'imagerie IRM ou en substitution de celui-ci pour les mesures de déplacements du tissu. Un système d'imagerie ultrasonore, appelé échocardiographie, est basé sur l'émission d'ondes acoustiques dans l'organisme. Lesdites ondes sont réfléchies différemment selon le type de structures anatomiques rencontrées. Le signal recueilli, correspondant aux échos des ondes émises, permet de reconstituer une image d'une partie de l'anatomie d'un patient ou d'un animal.

Selon un autre mode de réalisation, l'acquisition d'une image est réalisée au moyen d'un système d'imagerie à rayons X. Ce système d'imagerie est basé sur l'émission de rayons X sur les tissus. La mesure de l'atténuation des rayons X par les tissus permet de reconstituer des images en 2D ou 3D des structures anatomiques comme par exemple le coeur. Ce système peut être utilisé en complément d'un système d'imagerie IRM ou en substitution de celui-ci pour les mesures de balistique, c'est-à-dire de la position de la zone focalisée. La zone ciblée Z_{C} peut être éventuellement définie à partir du système d'imagerie.

Selon un autre mode de réalisation, l'acquisition d'une image est réalisée au moyen d'un système d'imagerie par émission de positons (TEP), appelé tomoscintigraphie. Ce système d'imagerie est basé sur la détection de radiations gamma émises par une substance radioactive injectée en faible quantité dans l'organisme, ce qui permet d'acquérir des images en coupe de certains organes comme par exemple le coeur. Ce système peut être utilisé en complément d'un système d'imagerie IRM ou en substitution de celui-ci pour les mesures de balistique, c'est-à-dire de la position de la zone focalisée et/ou de la zone ciblée. La zone ciblée Z_{C} peut être éventuellement définie à partir du système d'imagerie.

Les systèmes d'imagerie peuvent être, au besoin, couplés à des agents de contraste injectés ou ingérés, de manière à améliorer la visualisation des organes explorés.

### Système de positionnement

Un système de positionnement est utilisé lors de l'exécution des procédés de l'invention pour réaliser différents fonctions :
▪ une première fonction est de définir la position d'une zone ciblée Z_{C} que l'on souhaite atteindre. A cet effet, selon un exemple, le système de positionnement peut être couplé à un système d'imagerie pour récupérer sur l'image acquise ACQ_IMG la position d'une zone ciblée identifiée Z_{C}.
▪ une seconde fonction est de calibrer la balistique de chaque dispositif ou système réalisant une fonction des procédés de l'invention. Ainsi, les systèmes d'imagerie et le réseau de phase peuvent être calibrés en position avec un système de positionnement.
▪ Une troisième fonction est d'asservir la position de la zone focalisée Z_{F} sur la position de la zone ciblée Z_{C}. Pour cela une consigne est générée vers le réseau de phase pour que le faisceau soit défléchi correctement.
▪ Une quatrième fonction est de générer une consigne d'activation ou de désactivation des éléments du réseau de phase en fonction de la position projetée de la paroi transcostale dans le plan du réseau de phase. Cette fonction peut être assurée directement par le système d'imagerie vers le réseau de phase. L'utilisation du système de positionnement peut permettre d'améliorer la vitesse d'asservissement sans à avoir à être limité par les temps d'acquisition de l'image. Dans ce dernier cas, les calculs des positions de l'image projetée de la paroi transcostale peuvent être mémorisés dans le système de positionnement et les changements de positions du patient peuvent être calculés à partir d'une position de référence.

Selon un mode de réalisation, le système de positionnement comprend des capteurs ultrasonores extracorporels et un émetteur extracorporel dont les émissions de signaux ultrasonores sont réfléchies et détectées par les capteurs. Une position donnée ACQ_POS du coeur peut être obtenue par triangulation. Quatre capteurs des signaux permettent d'obtenir une bonne précision des positions de zones dans le coeur. Un avantage de cette solution est que l'asservissement d'une position de l'espace peut être plus rapide que par l'utilisation d'un système d'imagerie. En effet, cette solution nécessite moins de données à acquérir et à traiter. Les récepteurs acquièrent les variations sur une ligne de l'espace 3D.

Selon une variante de réalisation, l'émetteur est un émetteur dédié au système de positionnement. Selon une autre variante, l'émetteur peut être par exemple un élément du réseau de phase notamment pour établir la calibration de position de départ.

Les récepteurs du réseau de phase peuvent être utilisés. On préférera pour améliorer les précisions des mesures des capteurs d'ondes ultrasonores dédiés au système de positionnement.

Dans un autre mode de réalisation, le système de positionnement est réalisé au moyen de capteurs positionnés sur la peau.

Lorsque la position de la zone focalisée Z_{F} ou de la zone ciblée Z_{C} est déterminée à partir d'un système d'imagerie IMG, selon un exemple de réalisation, elle peut être reconnue automatiquement à partir d'un traitement d'images. A cet effet, un indicateur basé sur des variations de paramètres relatifs aux pixels de l'image peut être généré pour reconnaître automatiquement une zone spécifique du coeur. Selon un autre exemple de réalisation, la zone ciblée Z_{C} peut être identifiée au moyen d'un outil de traitement d'images avec l'usage d'une souris ou d'un pointeur graphique. La zone ciblée Z_{C} peut également être désignée par la définition de coordonnées de l'espace d'une image bidimensionnelle ou tridimensionnelle par exemple à partir d'un logiciel configuré pour piloter le réseau de phase.

Selon un mode de réalisation, la position de la zone ciblée Z_{C} peut être déterminée au moyen d'un cathéter intracardiaque comportant une sonde ultrasonore ou électromagnétique introduite dans un ventricule ou une oreillette du coeur et couplée respectivement à un ou des capteurs de position à ultrasons ou à un ou plusieurs capteurs de position électromagnétiques. Ledit système de positionnement peut donc être associé avec un système d'imagerie IRM ou au moins une sonde ultrasonore ou encore au moins un capteur intracardiaque. Une sonde ultrasonore permet, selon un mode de réalisation, de définir la zone ciblée Z_{C} pour asservir la position du faisceau focalisé et donc de la zone focalisée Z_{F}. Ce système peut être utilisé en complément d'un système d'imagerie IRM ou en substitution de celui-ci pour les mesures de déplacements du tissu.

Le système de positionnement permet de définir une position de la zone ciblée Z_{C} qui est transmise au réseau de phase RES_US ou au générateur de signaux ultrasonores pour l'asservir en position. En second lieu et éventuellement, le système de positionnement est capable de récupérer la position de la zone focalisée Z_{F} pour en déduire un écart avec la consigne de position de la zone ciblée. Le système de positionnement peut donc être intégré à un système d'imagerie par une fonction implémentée dans un calculateur ou être externe au système d'imagerie et y être associé pour extraire des données de l'image acquise.

Selon un mode de réalisation, le procédé de l'invention permet d'identifier une ou plusieurs zones ciblées Zc. Cette étape permet de réaliser différentes fonctions dont l'asservissement de la zone focalisée Z_{F}, comme décrit précédemment.

Une fonction possible du procédé de l'invention comprend une comparaison des données d'imagerie de la zone ciblée Zc ou d'une zone à sa proximité avant et après la stimulation cardiaque pour déterminer un paramètre de déformation tissulaire et/ou un paramètre de variation de températures. En effet, lorsqu'une comparaison d'images est réalisée, le procédé de l'invention permet d'identifier un gradient de valeurs sur l'image, c'est-à-dire une variation traduisant par exemple un déplacement du tissu cardiaque et/ou une variation de températures. C'est notamment le cas, lorsque le système d'imagerie est un système d'imagerie IRM et qu'il est couplé à un calculateur K₁. Après l'application d'un champ magnétique, ce dernier permet de déduire un paramètre de déphasage comprenant une valeur relative au déplacement des tissus et une valeur relative à la variation de températures. Le calculateur K1 permet d'effectuer notamment des opérations de traitement d'images pour déduire des paramètres de contrôle.

### Réseau de phase, générateur de signaux

Une troisième étape, notée GEN F_{US}, comprend la génération d'un ou d'une pluralité de faisceau(x) de signaux ultrasonores F_{US} focalisé(s) dans la ou les zone(s) ciblée(s) Z_{C}.

La sonde thérapeutique à ultrasons est montée sur un système mécanique de positionnement 3D permettant de localiser la sonde à l'aplomb de la région cardiaque ciblée. Grâce à la technologie réseau de phase, le faisceau ultrasonore peut être défléchi électroniquement autour de la position naturelle du point focal pour un réglage fin de la zone de tir. Cela permet de cibler aisément différentes régions du coeur pour stimuler plusieurs zones distinctes avec un délai temporel maîtrisé entre chaque tir.

Selon un exemple de réalisation, un réseau de phase à 256 éléments peut être utilisé avec une fréquence centrale à 1 MHz. Le foyer géométrique peut être configuré à 13cm.

Dans un mode de réalisation, le réseau de phase RES_US de signaux ultrasonores focalisé F_{US} comprend un ensemble d'éléments, tels que transducteurs élémentaires. La configuration du réseau de phase RES_US permet l'activation ou la désactivation des éléments, et le paramétrage des phases de chaque signal qui permet de piloter la déflexion du faisceau généré. La position du point focal du faisceau est donc déterminée par le paramétrage des phases de chaque signal définissant une déflexion donnée.

La configuration du réseau de phase permet de prendre en compte des obstacles entre les éléments du réseau de phase RES_US et le coeur tels que des côtes ou d'autres organes. Ainsi, il est possible de configurer la génération d'un ou de plusieurs faisceau(x) tout en évitant les obstacles. Cela permet, par exemple, de ne pas endommager les côtes d'un patient. Le procédé de stimulation de l'invention est particulièrement efficace lorsque le réseau RES_US est positionné en vis-à-vis de cage thoracique, au plus proche du coeur. Dans cette configuration, le procédé de l'invention permet d'établir une configuration du réseau de phase RES_US afin d'éviter d'insonifier des côtes, des brûlures pouvant être induites par absorption d'ondes ultrasonores émises dans une paroi osseuse.

Cette configuration peut être réalisée lors de la calibration d'un faisceau focalisé ou lors de l'opération de stimulation d'au moins une zone du coeur. La déflexion du faisceau peut être asservie pour compenser des mouvements respiratoires ou des contractions du coeur induisant un déplacement de la zone ciblée. Une nouvelle position d'une zone ciblée peut être déterminée par une estimation du déplacement de cette zone grâce à un système de positionnement tel que détaillé précédemment.

Les zones ciblées Z_{C} définissent une région dans le référentiel du coeur et sont donc susceptibles de se déplacer dans un référentiel terrestre. Le réseau de phase doit donc compenser les mouvements du coeur induits par les mouvements respiratoires ou les contractions du coeur. Ceci est réalisé grâce à l'asservissement de la position focalisée Z_{F} sur la position ciblée Z_{C}.

En particulier, selon un mode de réalisation, la déflexion du faisceau est asservie sur un paramètre de compensation des mouvements respiratoires du coeur. En effet, les mouvements de respirations génèrent des déplacements de la zone ciblée Z_{C} tout au long du cycle cardiaque et sont préférentiellement compensés lors de l'exécution des procédés de l'invention.

Eventuellement, selon un mode amélioré, la déflexion du faisceau peut être asservie sur un paramètre de compensation des mouvements de contraction du coeur survenant lors du complexe QRS. Une alternative est d'éteindre automatiquement le faisceau pendant l'apparition du complexe QRS pour éviter, par exemple, lors du procédé de stimulation de brûler ou de stimuler une zone connexe à la zone ciblée Z_{C}. Dans ce cas, l'extinction du faisceau est asservie sur le rythme de l'ECG pour que les moments d'apparition de la contraction et de l'extinction du faisceau soit synchrones.

Dans un mode de réalisation, le faisceau de signaux ultrasonores focalisé F_{US} est configuré pour générer une impulsion sur une zone ciblée Z_{C} de sorte à stimuler les tissus cardiaque dans cette zone. Le procédé de l'invention permet d'observer la réponse électrique et/ou la déformation tissulaire et/ou la température et/ou un niveau de cavitation dans ou à proximité de ladite zone. Lorsque la stimulation est réalisée sur une unique zone ciblée Z_{C}, cela permet, par exemple, de tester l'activité électrique de ladite zone.

Le réseau de phase RES_US peut également être utilisé comme dispositif ultrasonore de positionnement pour calibrer la position d'une zone focalisée Z_{F} avec un système d'imagerie IMG. D'autres applications sont possibles et décrites ci-après.

### Multi-faisceau(x)

Dans un mode de réalisation, le réseau de phase RES_US est configuré en phase pour générer une pluralité de faisceaux focalisés et donc générer une pluralité d'impulsions sur plusieurs zones ciblées Z_{C}. Il est alors possible de stimuler lesdites zones et observer les réponses électriques et/ou les déformations et/ou les températures et/ou les niveaux de cavitation desdites zones et ainsi d'effectuer éventuellement une comparaison des réponses. Lorsque la stimulation est réalisée sur une pluralité de zones ciblées Z_{C}, cela permet, par exemple, de calibrer les niveaux des signaux en différentes zones pour planifier des opérations de stimulation. Selon un autre exemple, la stimulation multipoints engagée simultanément permet d'effectuer une opération de resynchronisation des ventricules ou des oreillettes ou encore de modifier la fréquence de battements du coeur. D'autres applications sont possibles.

### Définition des signaux

Dans tous les modes de réalisation, le faisceau de signaux ultrasonores focalisé F_{US} est généré à une amplitude et à une durée configurées de manière à contrôler les niveaux de dommages mécaniques et/ou thermiques sur le tissu cardiaque.

Dans le cadre d'une application de stimulation cardiaque, on cherche une configuration permettant la meilleure stimulation et le moindre dommage des tissus. Une calibration des signaux selon le procédé de contrôle de l'invention peut être réalisée pour préparer une phase de stimulation dans laquelle les signaux définissent un ou plusieurs faisceaux optimisés. L'optimisation est effectuée en calibrant l'amplitude, la durée et la répétition des impulsions du faisceau focalisé. La calibration dépend de différents critères : âge, corpulence du patient, taille du coeur, épaisseur des tissus cardiaques, myocarde, zones stimulées, etc. la calibration des signaux permet donc d'adapter un faisceau pour définir une stimulation efficace.

Dans un mode de réalisation, le réseau de phase de signaux ultrasonores comprend un ensemble d'éléments, tels que transducteurs élémentaires. La configuration du réseau de phase permet l'activation ou la désactivation des éléments, et le paramétrage des phases de chaque signal qui permet de piloter la déflexion du faisceau. La position du point focal du faisceau est donc déterminé par le paramétrage des phases de chaque signal définissant une déflexion donnée.

La configuration du réseau de phase permet de prendre en compte des obstacles entre les éléments du réseau et le coeur tels que des côtes ou d'autres organes. Ainsi, il est possible de configurer la génération d'un ou de plusieurs faisceau(x) tout en évitant les obstacles. Cela permet, par exemple, de ne pas endommager les côtes d'un patient. Le procédé de stimulation de l'invention est particulièrement efficace lorsque le réseau est positionné en vis-à-vis de cage thoracique, au plus proche du coeur. Dans cette configuration, le procédé de l'invention permet d'établir une configuration du réseau de phase afin d'éviter d'insonifier des côtes pour éviter toute brûlure induite par l'absorption de l'onde ultrasonore émise dans une paroi osseuse.

Cette configuration peut être réalisée lors de la calibration d'un faisceau focalisé ou lors de l'opération de stimulation d'au moins une zone du coeur.

Le procédé de l'invention permet de configurer deux modes de stimulation cardiaque qui peuvent être utilisés :
▪ soit pour calibrer les signaux en vue d'une application spécifique, notamment en générant une impulsion et en analysant la réponse associée,
▪ soit de stimuler directement une zone ciblée pour obtenir un effet physiologique par génération d'un train d'impulsions.

Dans un premier mode de réalisation, l'amplitude d'une ou de plusieurs impulsions est inférieure à un premier seuil d'amplitude donné et la durée de la ou desdites impulsions est supérieure à un seuil de durée donné.

Ce premier mode est particulièrement avantageux pour stimuler une zone ou des zones du coeur en contrôlant que les dommages mécaniques n'apparaissent pas sur les tissus. Ce mode autorise un échauffement contrôlé de la zone ciblée Z_{C} sans toutefois causer de dommages thermiques. Les dommages thermiques sont limités en contrôlant la durée d'application du faisceau. Ce premier mode concerne plus particulièrement les impulsions représentées à la figure 6A.

Dans un second mode de réalisation, l'amplitude d'une ou de plusieurs impulsions est supérieure à un second seuil d'amplitude donné et la durée de la ou des impulsions est inférieure à un second seuil de durée donné.

Ce second mode est particulièrement avantageux pour stimuler une zone ou des zones du coeur en contrôlant que les dommages thermiques n'apparaissent pas sur les tissus. Les dommages thermiques n'apparaissent pas du fait que l'impulsion, si elle est suffisamment courte, ne provoque pas d'échauffement de la zone. La contrainte est relâchée sur les dommages mécaniques pouvant survenir avec des impulsions de plus grandes amplitudes que dans le premier mode, c'est-à-dire dont l'intensité de la force de radiation générée par le faisceau d'ultrasons est plus importante que celle du premier mode. Dans ce cas, le procédé de contrôle de l'invention permet de déterminer un seuil d'amplitude pour lequel l'impulsion n'est pas génératrice de dommages mécaniques des tissus de la zone focalisée ou d'une zone voisine.

Ce premier mode concerne plus particulièrement les impulsions représentées à la figure 6B.

Selon un exemple de réalisation, le premier seuil d'amplitude est identique au second seuil d'amplitude : A_{destru} et le premier seuil de durée est identique au second seuil de durée : T_{destru}. Les seuils sont représentés aux figures 4 et 5A, 5B dans lesquelles des uniques seuils respectivement en température T_{destru} et en amplitude A_{destru} sont représentés.

Selon un mode de réalisation, le procédé de l'invention comprend une étape d'injection d'un agent de contraste ultrasonore dans l'organe, c'est-à-dire le coeur.

Selon un premier mode de réalisation, l'agent de contraste ultrasonore permet de visualiser les faisceaux ultrasonores et de suivre le traitement par un système d'imagerie ultrasonore. Selon ce mode de réalisation, l'agent de contraste ultrasonore peut être utilisé en combinaison avec un dispositif d'imagerie ultrasonore de la même manière qu'un agent de contraste est utilisé pour l'amélioration de la visualisation des images IRM. Cet usage permet par exemple de détecter les zones saines et les zones pathologiques.

Selon un second mode de réalisation, l'agent de contraste ultrasonore peut être utilisé pour favoriser l'action du faisceau focalisé ultrasonore. La présence de l'agent de contraste permet d'améliorer l'apparition d'un phénomène de cavitation lorsqu'un signal est focalisé par la génération de microbulles. L'apparition de ce phénomène étant favorisé, une conséquence est de réduire le seuil des maximas d'amplitude des signaux acoustiques favorisant la stimulation d'un tissu dans une zone focalisée. Dans ce contexte, l'agent de contraste permet de réduire les seuils minimaux des niveaux de signaux ultrasonores utilisés pour stimuler une zone du coeur. L'agent de contraste ultrasonore permet donc de diminuer les puissances émises par le générateur de faisceaux en obtenant un résultat équivalent qu'avec une puissance plus forte sans agent de contraste ultrasonore.

Un intérêt de cette solution est de diminuer les puissances émises et donc de réduire les risques de dommages des tissus sollicités par les impulsions ultrasonores. Un autre intérêt est de diminuer les dommages causés sur d'autres organes ou sur des os par des émissions du faisceau.

Un dispositif pouvant être utilisé peut être par exemple celui nommé « SonoVue » de Bracco.

L'administration de l'agent de contraste ultrasonore peut être injectée dans une large gamme de valeurs. Des tests effectués sur des coeurs de cochons ont permis de valider l'amélioration de la génération d'une stimulation avec des puissances de pression acoustique plus faibles lorsqu'un agent de contraste ultrasonore était utilisé. Ces données sont d'un même ordre de grandeur que celles pouvant être obtenues chez l'homme. A titre d'exemple, une injection allant de 0,03 ou 0,20 ml/kg de SonoVue peut être utilisée pour obtenir un effet d'amélioration de l'effet de stimulation des tissus par application d'un faisceau focalisé. Une valeur de 0,1 ml/kg aboutit à un résultat concluant selon des tests conduits avec des signaux acoustiques focalisés produisant une amplitude de 2 à 15MPa sur des périodes d'impulsion inférieures à 80ms.

Dans ce test, la demi-vie d'élimination terminale a été de 12 minutes (allant de 2 à 33 minutes).

L'utilisation d'un agent de contraste ultrasonore permet d'obtenir des réductions de puissances nécessaires pour générer une stimulation dans la zone focalisée allant de quelques centièmes de MPa à quelques MPa. Les gains obtenus dépendent de la quantité d'agent de contraste ultrasonore administrée, de la période à laquelle le faisceau est émis après l'administration de l'agent de contraste et de données physiologiques de l'organe propre au patient ou à l'animal.

Un autre avantage est que l'effet de l'agent de contraste ultrasonore est indépendant de la durée de l'impulsion du faisceau, ce qui garantit un mode opératoire commun, c'est-à-dire avec les mêmes niveaux de puissances, pour différentes impulsions générées pendant la période pendant laquelle l'agent de contraste produit un effet.

### Contrôle de la température

Le procédé de l'invention permet de contrôler certains paramètres tels que la température ACQ_T en une ou plusieurs zones du coeur pour contrôler lesdites zones lors des opérations de calibration ou de stimulation. Un afficheur AFF_2 permet dans un mode de réalisation de visualiser, en coupe, en tranche ou d'une zone 3D, les températures du coeur ou d'au moins une région particulière de ce dernier.

La figure 1 représente un unique afficheur AFF_2 mutualisant les affichages de différents équipements dont l'affichage des images acquises. Selon un autre mode de réalisation, différents afficheurs peuvent être utilisés et dédiés à chaque équipement dans les procédés de l'invention.

Un calculateur, noté K₁, permet d'extraire les données acquises par imagerie IMG, telle qu'une IRM, pour déduire les températures dans certaines zones du coeur. Une extraction de la position de la zone ciblée ou de la zone focalisée peut être réalisée pour identifier la température dans cette zone ou à proximité. Par exemple, le calculateur K₁ est capable de calculer des variations de températures par une analyse des données de l'image acquise et d'identifier automatiquement les zones pour lesquelles les variations dépassent un seuil de température. Les positions des zones focalisées peuvent donc être déduites automatiquement par un traitement des données de l'image acquise en IRM.

Lorsque les variations de températures sont visibles à l'écran, un code couleur peut aider un opérateur à prévenir des hausses importantes de températures.

Selon un premier mode de réalisation, le contrôle de la température peut être réalisé lors de l'exécution du procédé de contrôle de l'invention pour calibrer le faisceau focalisé utilisé lors de l'opération de stimulation.

Selon un second mode de réalisation, le contrôle de la température peut être réalisé lors de l'exécution du procédé de stimulation pour application thérapeutique. Dans ce cas on parle d'un « contrôle actif ». Ce contrôle actif permet notamment de surveiller l'échauffement de la zone ciblée ainsi que des zones adjacentes à la zone ciblée.

### Contrôle de l'activité électrique

Dans un mode de réalisation, une mesure de l'activité électrique, notée ACQ_AE, peut être effectuée pendant et/ou après la génération du faisceau de signaux ultrasonores focalisé GEN F_{US} en complément de la mesure de la déformation tissulaire et/ou de la température et/ou d'un niveau de cavitation de manière à vérifier l'efficience de la stimulation ultrasonore dans ou à proximité de la zone focalisée. L'activité électrique ACQ_AE peut être mesurée localement à partir d'un cathéter ou à partir du système électrique permettant d'obtenir l'ECG.

Les temps de réponse électrique et/ou le niveau d'activité électrique dans ou à proximité de la zone focalisée peuvent être mesurés à partir d'un équipement électrique dédié noté SYS_ELEC_2 et représenté à la figure 1. Il permet d'acquérir le signal ACQ_AE traduisant un niveau d'activité électrique local. Un calculateur K2 peut être adjoint au système. Il permet la mise en oeuvre d'un mode de l'invention, en comparant un niveau d'activité électrique à un niveau de référence ou encore de rapprocher les valeurs de niveau d'activité électrique avec la mesure de déformation tissulaire ACQ_DEF. Ces corrélations / comparaisons de valeurs sont représentées par la fonction COMP de la figure 1. Un message d'état VERIF _2 permet d'aboutir au résultat de la comparaison. Selon un autre mode de réalisation, les fonctions réalisées par le calculateur K2 peuvent être assurées par le calculateur K1.

Selon un mode de réalisation, le niveau d'activité électrique peut être mesuré à partir d'un cathéter électrique introduit dans une région du coeur : ventricule ou oreillette.

Selon d'autres modes de réalisation, d'autres équipements peuvent être utilisés permettant de mesurer une activité électrique localement dans ou à proximité de la zone focalisée.

Notons qu'une activité électrique ACQ_AE locale dans la zone ciblée peut être contrôlée et déduite de l'ECG obtenu par exemple au moyen des électrodes. Dans ce dernier cas de figure, les systèmes électriques SYS_ELEC_1 et SYS_ELEC_2 de la figure 1 peuvent ne former qu'un seul équipement. Notamment, lorsqu'une stimulation est générée par le procédé de stimulation de l'invention et qu'une dépolarisation globale d'un ventricule ou d'une oreillette a lieu, alors l'activité électrique induite par le procédé de stimulation est visible sur un ECG par la présence d'un pic. Dans ce cas, un cathéter électrique peut éventuellement être utilisé pour corréler les mesures d'activités électriques déduites de l'ECG ou pour obtenir des mesures plus précises.

Dans un mode de réalisation, une impulsion est appliquée sur une zone ciblée Z_{C} du coeur pendant la repolarisation du tissu cardiaque. Une mesure de l'activité électrique du coeur et/ou de la déformation tissulaire et/ou d'un niveau de cavitation et/ou de la température induites par l'impulsion dans la zone ciblée Z_{C} du coeur est ou sont effectuée(s). Selon la réponse électrique, la déformation et/ou la température mesurées et/ou le niveau de cavitation, une calibration du faisceau de signaux ultrasonores F_{US} est réalisée. Différentes valeurs d'amplitudes et de durées de l'impulsion peuvent être configurées tout en contrôlant que la température n'excède pas localement un seuil au-delà duquel des dommages sur les tissus peuvent survenir.

Dans une application pour la resynchronisation cardiaque, ce contrôle permet de déterminer des positions de placement de sondes ayant de bonnes propriétés de réponses électriques. Les sondes sont destinées à être fixées sur la paroi du myocarde et à générer des impulsions cardiaques en une pluralité de points du coeur d'un patient. Les impulsions ainsi générées assurent la synchronisation des ventricules ou des oreillettes du coeur.

Lorsque la déformation du tissu cardiaque ou l'activité électrique de la zone focalisée est inférieure à un seuil prédéterminé pour une amplitude et une durée données d'au moins une impulsion du faisceau focalisé F_{US}, la zone focalisée est alors considérée comme « non répondante ».

Selon un premier contrôle, lorsque la zone focalisée est considérée comme non répondante, le procédé de contrôle de l'invention permet par exemple d'ajuster en durée et en amplitude un nouveau faisceau focalisé pour effectuer une seconde mesure de calibration.

Dans ce cas, le procédé de calibration d'un faisceau focalisé permet de définir un nouveau faisceau focalisé en prenant notamment en compte les variations de températures afin d'éviter d'endommager les tissus avec le nouveau faisceau appliqué dans la zone focalisée.

Selon un second contrôle consécutif à une opération d'ablation, lorsque la zone focalisée est considérée comme non répondante, le procédé de contrôle permet, par exemple, de vérifier qu'une ablation d'une zone génératrice d'une arythmie s'est bien déroulée.

Il est possible de déduire un niveau d'activité électrique théorique généré en fonction d'un niveau de déformation des tissus mesuré. Ainsi, selon un mode de réalisation de l'invention, le procédé de contrôle et de stimulation ne nécessitent pas de mesure de l'activité électrique puisqu'elle peut être déduite d'une mesure de déformation des tissus. Les procédés de l'invention peuvent donc avantageusement être non invasifs.

Le procédé de calibration peut comprendre une étape d'étalonnage permettant d'établir une règle de correspondance entre les niveaux d'activités électriques et les niveaux de déplacements des tissus mesurés lors de l'application d'un faisceau focalisé dans une zone focalisée donnée. Cet étalonnage peut également prendre en compte les réponses aux températures dans cette zone.

Lors du procédé de stimulation, cet étalonnage peut permettre de mesurer uniquement les niveaux de déformation des tissus et déduire l'activité électrique induite.

Lorsque la mesure de la déformation des tissus, ou d'une caractéristique propre à leur élasticité, est faible, voire nulle, alors il est possible de déduire qu'aucune activité électrique n'est générée.

### Contrôle contraction cardiaque

Enfin, le procédé de stimulation de l'invention peut comprendre une étape de vérification de la présence d'une contraction cardiaque. La vérification d'une contraction mécanique peut être effectuée au moyen d'une sonde de pression sanguine par exemple par une mesure du pouls ou par une sonde intra-aortique pour mesurer la contraction du coeur. Cet équipement de mesure de contraction mécanique est noté CAP_CONTRACT sur la figure 1 et la mesure est notée ACQ_CONTRACT.

Le contrôle d'une contraction cardiaque est particulièrement intéressant pour une application du procédé de l'invention avec un dispositif mobile ou portable, par exemple intégré à un camion de pompiers. Le dispositif portable de l'invention dans ce mode de réalisation ne comprend pas de système d'imagerie. En revanche, le dispositif mobile permet de mesurer la présence d'une contraction du coeur pour vérifier qu'une ou plusieurs stimulations appliquées sur un patient ont bien généré un effet physiologique.

La figure 2 illustre un exemple d'une séquence d'un ECG du coeur. La séquence de l'électrocardiogramme représentée comprend deux contractions 1 et 2 du myocarde.

L'onde P qui commence à l'instant 20 correspond à la dépolarisation des oreillettes, suivie par le complexe QRS qui comprend la dépolarisation des ventricules qui commence à l'instant 21 et la repolarisation des oreillettes qui est confondue avec le complexe QRS et enfin l'onde T correspondant à la repolarisation des ventricules à l'instant 13.

### Contrôle de la cavitation

Selon un mode de réalisation, le procédé de contrôle ainsi que le procédé de stimulation de l'invention comprennent une étape de calcul d'un niveau de cavitation dans la zone focalisée. Le phénomène de cavitation, repose sur le phénomène de création de bulles dans la région focalisée générée par la vibration des ondes ultrasonores émises.

On distingue deux cavitations pouvant survenir :
▪ une cavitation stable qui correspond à un phénomène de création de bulles dans la zone focalisée ou à proximité. Les bulles favorisent éventuellement le déplacement des tissus;
▪ une cavitation inertielle qui est dans la continuité de la cavitation stable dans laquelle les bulles se vaporisent ou éclatent pouvant créer des dommages sur les tissus mais pouvant également stimuler la zone. Le phénomène de cavitation inertielle est créé par génération d'une pression négative localement au-delà d'un certain seuil.

Le phénomène de cavitation peut être déterminé par un dispositif de mesure d'un niveau de cavitation. Selon un exemple de réalisation, il peut s'agir d'un dispositif ultrasonore pour la détection d'un niveau de cavitation ACQ_CAV dans ou à proximité de la zone focalisée, noté SYS_CAV sur la figure 1. Ce dispositif comprend un capteur ou plusieurs capteurs d'ondes ultrasonores et un calculateur effectuant une analyse spectrale ACQ_US des ondes ultrasonores réfléchies dans ou à proximité de la zone focalisée.

Selon un mode de réalisation, un calculateur K1 permet de centraliser les différentes mesures effectuées par différentes équipements dont les mesures de niveau de cavitation ACQ_CAV, de déformation tissulaire ACQ_DEF, de température ACQ_T. Les mesures sont éventuellement comparées à des seuils. Des alarmes peuvent être générées selon les dépassements vis-à-vis des seuils prédéfinis de ces mesures en temps réel. Un afficheur AFF_2 permet éventuellement d'afficher ces valeurs et les images acquises par les différents équipements.

Selon un autre mode de réalisation, chaque équipement peut être couplé à un calculateur dédié et un afficheur dédié.

Plus le spectre est étalé, c'est-à-dire que le bruit est important dans ou à proximité de la zone focalisée, plus le phénomène de cavitation est déterminé comme important. Des seuils peuvent être définis et déterminés à partir du procédé de contrôle de l'invention pour étalonner les valeurs d'amplitudes et de durées des signaux utilisés notamment pour la stimulation pour différentes applications cardiaques.

Selon un mode de réalisation, le contrôle effectué lors de l'exécution d'un procédé de stimulation détecte en temps réel un niveau représentant l'importance du phénomène de cavitation. Le procédé de stimulation peut prendre en compte dynamiquement les niveaux mesurés :
▪ soit pour stopper le ou les faisceaux en cas de niveau trop important ;
▪ soit pour configurer automatiquement le niveau de pression générée au point focal par une consigne d'amplitude de durée d'impulsion.

Le phénomène de cavitation dans la zone ciblée peut être également mesuré par les procédés de l'invention :
▪ soit par extrapolation d'une déformation tissulaire essentiellement générée par ce phénomène ;
▪ soit par des capteurs à ultrasons extracorporels comme précisé précédemment par une analyse spectrale des signaux réfléchis ;
▪ soit par un capteur ultrasonore intracardiaque positionné à proximité de la zone ciblée.

Selon un autre mode de réalisation qui peut se combiner avec les précédents modes, une mesure d'un niveau de cavitation est comparée à un premier seuil et une mesure d'une déformation tissulaire est comparée à un second seuil.

Lorsque le niveau de cavitation dépasse le premier seuil un premier risque de dommages est identifié. Lorsque la mesure de la déformation tissulaire dépasse le second seuil, un second risque de dommages est identifié. Un algorithme permettant de prendre en compte les deux risques peut permettre de générer une consigne d'arrêt de la procédure de calibration ou de stimulation, par exemple en coupant automatiquement le signal ultrasonore émis. Un intérêt est de renforcer la procédure de sécurité encadrant une intervention visant à stimuler une zone du coeur ou à améliorer la détection d'un risque.

Enfin, un autre intérêt réside dans la possibilité d'obtenir un double indicateur dans le temps basé sur une double appréciation d'un risque par deux moyens différents. En effet, un capteur ultrasonore mesurant le niveau de cavitation peut acquérir entre 10 et 5000 signaux par seconde, alors qu'un capteur de déformation des tissus, utilisant par exemple l'IRM, nécessitera un taux de rafraichissement de son acquisition de 0,5 à 10 signaux par seconde. Il est donc possible de disposer d'un système comprenant des moyens de calculs permettant d'effectuer une première mesure du niveau de cavitation et lorsqu'un premier risque est identifié, alors une détection de l'évolution du niveau de déformation peut être analysée dans l'espace de temps succédant la détection du premier risque identifié. De ce fait, il est possible d'engendrer une coupure du signal focalisé le plus rapidement possible.

Par ailleurs, le premier dépassement du niveau de cavitation peut conduire à analyser l'évolution de la déformation tissulaire sans que pour autant le second seuil ne soit dépassé. L'analyse porte alors sur la tendance des tissus à se déformer et non sur le dépassement d'un second seuil donné. Ainsi, il est possible grâce au procédé de l'invention de couper la génération du signal le plus rapidement possible.

Selon une alternative, la mesure du niveau de cavitation n'est plus corrélée avec la mesure de déformation tissulaire mais avec l'activité électrique dans ou à proximité de la zone focalisée. Dans ce dernier cas, le même traitement peut être appliqué à ces deux mesures, notamment en ce qui concerne l'analyse des signaux par les différents équipements dans le temps.

L'algorithme peut également permettre la génération d'une coupure des signaux ultrasonores uniquement lorsque deux seuils sont dépassés, un premier seuil concernant le niveau de cavitation et un second seuil concernant, par exemple, la déformation des tissus. Cette autre option permet de considérer des erreurs de mesures d'un des appareils de mesure, tels que l'IRM ou un dispositif ultrasonore par exemple.

Le procédé de l'invention permet donc de définir différentes stratégies de contrôle de la stimulation d'une zone pour éviter de causer des dommages lors d'une telle opération. Une première stratégie peut correspondre à assurer un degré de sécurité maximal. Dans ce cas, un seul dépassement de seuil permet de générer une coupure du faisceau de signaux. Une seconde stratégie correspond à assurer l'élimination des erreurs de mesures. Dans ce cas, le procédé permet de confirmer qu'un dommage survient si deux seuils de mesures d'équipements différents sont dépassés.

Notons que le procédé s'applique avec une pluralité de mesures correspondant à des grandeurs mesurées avec différents équipements. Ainsi, une corrélation de trois types de signaux peut être effectuée en effectuant des comparaisons vis-à-vis de trois seuils. Par exemple, le niveau de cavitation est comparé à un premier seuil, une déformation tissulaire est comparée à un second seuil et une activité électrique est comparée à un troisième seuil. Une stratégie de contrôle peut être que le dépassement de deux seuils sur trois suffit à générer une coupure des signaux ultrasonores. Cette solution permet un compromis entre un gain de sécurité (2 appareils sur 3 ont détecté un risque) et une prise en compte d'erreurs de mesure (1 appareil sur 3 n'a rien détecté). D'autres possibilités peuvent être décidées selon si l'on souhaite une configuration favorisant une sécurité maximale : au moins un seuil est dépassé entraîne la coupure du faisceau ou selon si l'on souhaite une configuration dans laquelle on prend en compte des erreurs de mesures : les trois seuils doivent être dépassés pour engager une coupure du faisceau.

### Contrôle des déplacements tissulaires

La génération du ou des faisceau(x) de signaux ultrasonores GEN F_{US} permet de générer une poussée ultrasonore localisée dans une zone focalisée Z_{F} et possiblement sensiblement à proximité de cette zone par déplacement des tissus.

La poussée ultrasonore peut être accompagnée de phénomènes secondaires qui sont liés ou non à la génération de cette poussée. Un premier phénomène est lié à l'agitation moléculaire induite par le faisceau au point focalisé. Un second phénomène est lié à la cavitation induite par le faisceau focalisé.

Le phénomène de cavitation est à l'origine de la création de microbulles dans ou à proximité de la zone focalisée pouvant :
▪ soit améliorer l'effet de déplacement des tissus ou de la propagation de l'activité électrique induite par la contraction du tissu, on parle de cavitation stable ;
▪ soit détruire les tissus par génération d'une pression négative localement au-delà d'un certain seuil. Au-delà d'un seuil de pression négative, la destruction des bulles ou leur vaporisation crée ainsi des lésions sur les tissus, on parle dans ce cas de cavitation inertielle.

Le procédé de l'invention, selon la configuration des signaux ultrasonores, est capable de générer un déplacement des tissus en contrôlant favorablement les effets de cavitation pour éviter la destruction des tissus.

Un intérêt est que le phénomène de cavitation peut induire un stress mécanique appliqué au tissu en plus de la force de radiation ultrasonore pouvant induire une activité électrique.

Lorsque les impulsions sont générées pendant une durée inférieure à un seuil donné, correspondant à des impulsions de courtes durées, alors il est possible d'induire une activité électrique en minimisant l'échauffement de la zone focalisée.

Les déplacements des tissus peuvent être obtenus grâce à un système d'imagerie tel qu'un système d'imagerie IRM. Dans ce cas, un avantage est qu'un unique équipement permet d'obtenir les positions de la zone ciblée et de la zone focalisée, et une quantification des variations de températures et de déplacements des tissus.

Lorsque les mesures de températures et de déformations des tissus sont réalisées par un même équipement d'imagerie IRM, ce qui est possible grâce à la différence intrinsèque entre le déphasage dû à un changement de température et celui dû à un déplacement local, ces deux effets peuvent être discriminés et mesurés simultanément. Cette solution est donc avantageuse du point de vue du nombre d'équipements utilisés et de la simplification des calculs à mettre en oeuvre pour déduire une donnée de déformation tissulaire et une donnée de température à partir de la même acquisition.

Le couplage de ces deux équipements permet donc de suivre simultanément la température et le déplacement du tissu cardiaque par une seule méthode d'acquisition.

Enfin, la mesure de la déformation des tissus permet de calibrer les niveaux d'élasticité du tissu dans une zone ciblée pendant le procédé de contrôle, par exemple pour constater qu'une nécrose de coagulation est terminée. En outre, lorsque le procédé de contrôle permet de vérifier qu'une ablation s'est bien déroulée, une comparaison des niveaux d'élasticité pendant l'ablation avec une mesure réalisée préalablement permet de quantifier la proportion de zone ablatée en fonction d'un niveau à atteindre.

Les déformations tissulaires peuvent être mesurées à partir d'un système d'imagerie IRM comme précisé précédemment. D'autres systèmes d'imagerie peuvent être utilisés lorsque ces derniers permettent de contrôler une déformation ou une élasticité du tissu. Selon un autre mode de réalisation, les déformations tissulaires peuvent être mesurées par un cathéter comprenant une sonde à ultrasons introduite à proximité de la zone focalisée (Z_{F}) mesurant la déformation tissulaire. Eventuellement, un cathéter comprenant une sonde de pression peut être utilisé à proximité de la zone focalisée pour déduire des déformations tissulaires.

Selon un mode de réalisation, comme pour la mesure des variations de températures, le calculateur K₁ permet de récupérer la position de la zone ciblée en récupérant la consigne de position ou la position de la zone focalisée Z_{F} par un traitement d'images. L'étape VERIF_1 permet une récupération des données d'imagerie IMG par le système de positionnement SYS_POS. Les variations de déplacements des tissus peuvent donc être automatiquement calculées dans une région asservie sur la position récupérée. Le calculateur peut être le même que celui utilisé pour les autres calculs de paramètres ou un calculateur dédié à la mesure des déformations tissulaires. Une mesure de l'élasticité du tissu peut être déduite des déplacements/déformations de tissus.

En outre, sur la figure 1, l'étape VERIF_1 peut aussi se référer à une étape de contrôle de la calibration de la balistique entre le système d'imagerie IMG et le système de positionnement SYS_POS. Ce contrôle consiste à vérifier que la position d'un point de l'espace évaluée par un équipement corresponde bien à la position d'un point de l'espace évaluée par un autre équipement.

Selon un autre mode de réalisation, les mesures de températures et de déformations sont réalisées par des équipements différents. Les mesures de températures peuvent, par exemple, être réalisées par un système d'imagerie IRM et les mesures de déformation des tissus peuvent être effectuées par un système d'imagerie à ultrasons.

Lorsqu'un système d'imagerie est utilisé, il est possible de visualiser les déformations tissulaires ACQ_DEF localement sur une coupe 2D ou une tranche 3D de l'image acquise ou encore d'une région de l'image 3D au moyen d'un afficheur AFF_2. L'afficheur peut être le même que celui permettant de visualiser les températures de la région du coeur considérée ou un afficheur dédié.

### Applications / synchronisation avec ECG

Selon l'application, la stimulation par ultrasons est réalisée soit pendant la dépolarisation des oreillettes et/ou des ventricules soit pendant la repolarisation des oreillettes et/ou des ventricules.

Pour réaliser cette synchronisation, le faisceau de signaux ultrasonores focalisé F_{US} est synchronisé sur le rythme de l'ECG du coeur de sorte à sélectionner une fenêtre temporelle pendant laquelle la stimulation est réalisée lors de la repolarisation ou lors de la dépolarisation du tissu cardiaque suivant l'effet physiologique souhaité.

Par exemple, une application de la stimulation lors de la calibration concerne le contrôle de la balistique. Le contrôle de la balistique permet de s'assurer que la position de la zone ciblée correspondant bien à la position de la zone focalisée. En effet, avant l'utilisation du système pour effectuer une stimulation d'une zone du coeur, une calibration des positions des différents équipements permet un gain de précision du procédé. Ainsi, la calibration de la balistique permet de calibrer le système de positionnement du réseau de phase et celui qui est utilisé pour l'asservir, par exemple un système d'imagerie ou une sonde de position.

Dans l'application qui concerne la calibration de la balistique, le procédé de l'invention permet de générer une stimulation d'une zone focalisée après la dépolarisation des oreillettes et/ou des ventricules de manière à ne pas induire d'extrasystole. Le procédé de calibration de l'invention comprend donc une synchronisation du réseau de phase sur l'ECG de sorte à générer le faisceau focalisé à un moment précis du battement cardiaque.

Selon d'autres applications de stimulation comme la resynchronisation ventriculaire ou auriculaire, le réseau de phase est synchronisé, à un autre moment du cycle cardiaque, sur l'ECG. Dans ce cas, la stimulation est réalisée après la repolarisation des oreillettes et/ou des ventricules selon l'application que l'on souhaite réaliser. L'ECG permet donc de synchroniser le réseau de phase afin de stimuler au moment voulu le tissu cardiaque selon l'effet physiologique souhaité.

Pour les applications telles que la génération d'une fibrillation ou la génération d'une extrasystole, la stimulation est réalisée également pendant la repolarisation des oreillettes et/ou des ventricules. Pour la génération d'une fibrillation cardiaque, la stimulation est avantageusement générée lors de l'onde T de l'ECG.

La figure 3A est un diagramme représentant une impulsion, sur une zone ciblée Z_{C} du coeur pour le contrôle de ladite zone, d'un faisceau de signaux ultrasonores F_{US} d'amplitude A₁ et de durée D₁, la durée d'application totale étant appelée D_{SIGNAL}. Le procédé de contrôle de l'invention permet à partir d'une impulsion de calibration de définir les seuils de stimulations électriques sans causer de dommages aux tissus.

Les amplitudes Aᵢ et les durées Dᵢ des impulsions pour la stimulation de la zone ciblée Z_{C} du coeur sont choisies de manière à minimiser préférentiellement un dommage mécanique ou préférentiellement un dommage thermique.

La figure 3B représente la génération d'un faisceau focalisé comprenant une succession d'impulsions sur au moins une zone ciblée Z_{C} du coeur pour la stimulation de ladite zone. Les impulsions sont appliquées dans une région du coeur asservie sur la position d'une zone ciblée préalablement définie.

Les signaux de la figure 3B ont une amplitude A₂ pendant une durée D₂ et sont séparées par un intervalle de temps D₃, la durée d'application totale étant désignée D_{SIGNAL}. L'amplitude du signal peut représenter le niveau de pression acoustique induit dans la zone focalisé exprimé en Pascal. Selon un autre mode de représentation, l'amplitude du signal A₂ peut représenter l'amplitude du faisceau ultrasonore recomposé de manière cohérente dans la zone focalisée Z_{F}. L'amplitude du faisceau ultrasonore recomposé dans la zone focalisée et la pression acoustique induite par le faisceau dans la zone focalisée sont liées de sorte qu'il est indifférent d'évoquer l'une ou l'autre de ces notions pour décrire le signal de la figure 3B.

Dans un premier mode de réalisation, les impulsions sont configurées de sorte que la pression acoustique A₂ dans la zone focalisée Z_{F} soit comprise dans une gamme de pression de [2 - 8MPa] pour des durées d'impulsions D₂ comprises dans la gamme de durée [1 - 50ms]. Les impulsions générées sont synchronisées sur l'ECG du patient ou de l'animal. Ces gammes de valeurs sont particulièrement avantageuses pour provoquer une stimulation électrique tout en maintenant un seuil de dommage mécanique du tissu cardiaque inférieur à une valeur seuil. Ladite valeur seuil est calculée à partir d'un paramètre correspondant à une proportion donnée d'une contraction ou d'une relaxation ou d'une élasticité du tissu.

Dans un second mode de réalisation, les impulsions sont configurées de sorte que la pression acoustique A₂ dans la zone focalisée Z_{F} est comprise dans une gamme de pression de [8 - 12MPa] pour des durées d'impulsions D₂ comprises dans la gamme de durée [50µs - 1ms].

Ces gammes de valeurs sont particulièrement avantageuses pour provoquer une stimulation électrique tout en maintenant un seuil de dommage thermique du tissu cardiaque inférieur à une valeur seuil qui peut être, par exemple, une valeur seuil en température. Pour cela, un contrôle de la température permet de contrôler les possibles dommages thermiques pouvant survenir lors de la génération des impulsions. La température mesurée localement, par exemple grâce à l'IRM, peut indiquer un risque de dommage thermique pouvant survenir.

Les impulsions sont donc générées pour provoquer une stimulation électrique dans les tissus cardiaques tout en maintenant localement dans la zone ciblée (Z_{C}) la température inférieure à une température seuil (T_{MAX}).

Selon un mode de réalisation, la durée D₃ est avantageusement supérieure 200 ms. Selon un mode de réalisation, la durée D₃ est avantageusement comprise dans l'intervalle QT. Selon une configuration, une impulsion est générée à chaque cycle de battement cardiaque. Selon d'autres configurations, plusieurs cycles de battements cardiaques peuvent séparer la génération de chaque impulsion.

Dans tous les modes de réalisation, un contrôle actif des zones voisines de la zone focalisée peut être réalisé par une mesure de l'activité électrique et/ou une mesure de la déformation tissulaire et/ou une mesure d'un niveau de cavitation et/ou une mesure de la température des zones voisines de la zone ciblée Z_{C} du coeur de manière à ne pas endommager les zones voisines de la zone ciblée Z_{C}.

Dans tous les modes de réalisation, le procédé de stimulation peut comprendre un contrôle de la balistique qui peut être réalisé par exemple au moyen d'une impulsion du signal et d'un système d'imagerie. Ce contrôle pendant le procédé de stimulation peut permettre de vérifier que l'étalonnage de la position calibrée lors du procédé de calibration est bien maintenu. Dans ce cas, le contrôle comprend la vérification que la zone focalisée Z_{F} et la zone ciblée Z_{C} sont confondues ou sensiblement proches.

La figure 4 représente les diagrammes correspondant à l'évolution de la température 40, 41, de la déformation du tissu 47, 47' et du phénomène de cavitation 48 au niveau de la zone ciblée Z_{C} du coeur selon l'amplitude et la durée des impulsions 42, 43 d'un faisceau de signaux ultrasonores F_{US} sur ladite zone. Deux cas de figure sont représentés selon les gammes de valeurs envisagées définissant le faisceau focalisé.

Les courbes représentées sont indicatives pour certains points critiques. En revanche, les formes des tracés ne sont pas représentées fidèlement aux courbes obtenues lors de tests.

Les déformations tissulaires 47, 47' et le phénomène de cavitation 48,48' sont représentés sur les mêmes graphiques 44 et 45.

Selon un premier cas de figure, l'impulsion 42 appliquée sur la zone ciblée Z_{C} du coeur présente une amplitude A₁ comprise entre [2 MPa ; 8 MPa] pendant une durée D₁ comprise entre [1ms ; 50ms] Une plage de valeurs en amplitude de [4 MPa ; 8 MPa] permet d'obtenir une stimulation des tissus cardiaques en limitant considérablement les effets destructifs thermiquement et mécaniquement des tissus.

A l'issue de la durée D₁, la température 23 de la zone ciblée Z_{C} mesurée, notée T_{mes_1}, est proche de la température maximale T_{MAX} pour laquelle le tissu cardiaque peut être endommagé par brûlure. La déformation du tissu cardiaque 47 peut être observée. Avantageusement, le procédé de calibration permet d'évaluer la valeur de la durée D1 de l'impulsion pour laquelle Tₘₐₓ n'est pas dépassée.

Dans ce premier cas de figure, sont représentées, sur le graphique 51 des figures 5A et 5B, la probabilité PROB_DT que des dommages thermiques 52 surviennent et la probabilité PROB_DM que des dommages mécaniques surviennent localement sur les tissus de la zone focalisée en fonction de la durée D₁ d'application de l'impulsion pour une amplitude donnée A₁.

La courbe 52 illustre que plus la durée D₁ de l'impulsion est longue, plus la probabilité de survenance de dommages thermiques 52 devient importante.

Une durée seuil, notée D_{DT}, est indiquée au-delà de laquelle l'impulsion 42 génère un échauffement pouvant causer des dommages thermiques. Cette durée seuil D_{DT} est calculée pour une amplitude donnée de l'impulsion. Une plage de valeurs de durées PD est représentée pour lesquelles les dommages thermiques 52 sont limités.

Le même graphique représenté à la figure 5B représente le cas d'exemple lors que la durée D1 est inférieure à la durée seuil pour une amplitude d'impulsion donnée. Dans ce cas les dommages thermiques sont négligeables, voire nuls.

La durée d'application de l'impulsion peut donc être calibrée de sorte à ne pas dépasser une durée seuil pour laquelle l'augmentation de la température locale provoque des dommages thermiques sur les tissus. Cette température locale peut être mesurée grâce à un système d'imagerie, tel que l'IRM. Un calculateur récupérant la position de la zone ciblée Z_{C} peut extraire des données de l'IRM, telles que la température locale ou les variations de températures dans ou à proximité de la zone focalisée Z_{F}.

Un premier avantage de ce cas de figure est de pouvoir contrôler les dommages thermiques tout en générant une déformation des tissus induits par la poussée ultrasonore du faisceau. La poussée ultrasonore sur les tissus appliquée localement peut favoriser la survenance d'une dépolarisation globale du ventricule ou de l'oreillette obtenue par l'effet de contraction des tissus se propageant dans l'organe.

Un second avantage de ce cas de figure est de générer un faisceau focalisé dont l'amplitude de poussée ultrasonore est inférieure à un seuil dans ou à proximité de la zone focalisée. Ce seuil est noté sur les figures 4 et 5 : « A_{destru} ». Le seuil A_{destru} correspond au seuil d'amplitude de poussée ultrasonore du faisceau focalisé au-delà duquel des dommages mécaniques surviennent sur les tissus. Les dommages mécaniques sont issus du phénomène de cavitation favorisant la génération de microbulles localement qui sont détruites sous l'effet de la poussée ; ces dernières endommagent les tissus lors de leur destruction.

Ainsi, ce premier cas de figure permet de créer un faisceau focalisé dont l'amplitude des impulsions est maîtrisée pour ne pas causer de dommage mécanique sur les tissus cardiaques et dont la durée est également maîtrisée pour ne pas causer de dommage thermique.

Selon un second cas de figure, l'impulsion 43 appliquée sur la zone ciblée Z_{C} du coeur présente une amplitude A₁' comprise entre [8 MPa ; 12 MPa] pendant une durée D₁' comprise entre [50µs ; 1 ms]

A l'issue de la durée D₁', la température 23' de la zone ciblée Z_{C} mesurée T_{mes_2} est inférieure à un seuil de température critique notamment indiqué par la température maximale T_{MAX}. Un cas d'une telle impulsion est représenté à la figure 5B. L'impulsion étant de courte durée, c'est-à-dire inférieure à 1ms, les tissus ont peu de temps pour s'échauffer. La stimulation est essentiellement provoquée par un phénomène de cavitation. Les déformations des tissus 47' générées par la poussée ultrasonore sont faibles en deçà de 1 ms.

Ce cas de figure est intéressant du point de vue de l'absence de dommages thermiques des tissus qui ne peuvent survenir dans une durée d'impulsion inférieure à 1ms. La partie de la courbe 52' se prolongeant au-delà de T_{destru} sur la zone 59 est représentée en considérant une impulsion 43 qui dépasserait la durée D_{DT}. Dans ce cas de figure, des dommages thermiques surviendraient. On note qu'avant T_{destru}, les dommages thermiques 52' des figures 5A et 5B sont négligeables, voire nuls sur le graphique 54.

Dans ce cas de figure, du fait que la poussée ultrasonore est générée pendant une durée très faible, la viscoélasticité du tissu ne permet pas au tissu de générer une réponse mécanique à la stimulation.

Sur la figure 5A, pour une amplitude supérieure dans la gamme indiquée, on observe uniquement un phénomène de cavitation 48', c'est-à-dire une génération de microbulles exerçant un effet mécanique sur les tissus cardiaques. Sur la figure 4, on observe que la courbe 48, 48' représentant le phénomène de cavitation est dépendante du niveau d'amplitude de l'impulsion A₁, A₁'. En effet, le phénomène de cavitation 48' à droite de la figure 4 est plus important lorsque l'amplitude A₁' de l'impulsion est plus élevée que l'amplitude A₁ comme cela est représenté sur la figure 4 à gauche en comparant les courbes 48 et 48'.

La figure 5A représente une courbe 55 dans le graphique 54 représentant la probabilité PROB_DM que des dommages mécaniques surviennent pendant ou après l'application d'un faisceau focalisé lorsque le niveau d'amplitude de l'impulsion est supérieure à un seuil A_{destru}. On note que cette probabilité PROB_DM augmente avec l'augmentation de l'amplitude du faisceau focalisé comme illustré sur les courbes 55 des figures 5A et 5B. Les dommages des tissus sont essentiellement liés, dans ce cas d'exemple, à un phénomène de cavitation inertielle : les microbulles explosent ou se vaporisent et créent des lésions sur les tissus cardiaques. Il s'agit alors de dommages mécaniques. La période très courte d'application du faisceau ne permet pas une élévation de la température des tissus dans la zone focalisée.

Il existe une plage d'amplitudes PA, représentée sur la figure 5, du faisceau focalisé pour laquelle les dommages mécaniques 55 sont minimes, voire négligeables. Un cas favorable est représenté à la figure 5B dans lequel les dommages mécaniques 55 sont maintenus à très faibles niveaux.

Une génération d'un faisceau focalisé dont l'amplitude est inférieure à l'amplitude seuil A_{destru} permet de limiter les dommages mécaniques liés au phénomène de cavitation inertielle 42.

Dans ce second cas de figure, le procédé de l'invention permet de générer des impulsions dites « courtes » dont l'amplitude est dans une plage de valeurs permettant d'éviter le phénomène destructif de cavitation inertielle, ce phénomène pouvant causer des dommages mécaniques. Les impulsions courtes ont donc une durée inférieure à une durée au-delà de laquelle les dommages thermiques des tissus cardiaques peuvent survenir.

Un avantage lié au second cas de figure, est que le phénomène de cavitation peut entraîner une dépolarisation globale d'un ventricule ou d'une oreillette lorsqu'elle est appliquée dans une ou plusieurs zones ciblées.

Ce second cas de figure permet de stimuler le coeur électriquement sans causer de dommage par exemple avec une impulsion telle que représentée sur la partie droite de la figure 5B. La durée et l'amplitude des impulsions sont donc contrôlées et configurées de sorte à définir un ou plusieurs faisceaux focalisés pour la stimulation du coeur.

Dans tous les modes de réalisation, les mesures de la déformation tissulaire et de la température de la zone ciblée Z_{C} ou dans sa région proche peuvent être effectuées par un même système d'imagerie. Comme précisé précédemment, selon un mode de réalisation, ledit système d'imagerie peut être un système d'imagerie IRM. Selon un autre mode, deux équipements différents, comme par exemple par un système d'imagerie IRM et un système d'imagerie à ultrasons, peuvent être combinés pour déterminer la température et les déformations dans ou à proximité de la zone focalisée.

Les dommages thermiques pouvant survenir sur les tissus sont, par exemple, de type hyperthermie ou coagulation.

Les dommages mécaniques lorsqu'ils sont induits par le phénomène de cavitation peuvent être par exemple : une volatilisation, une vaporisation ou encore un éclatement des bulles causant des lésions sur les tissus.

La figure 6A représente un premier faisceau focalisé 50 appliqué dans une zone ciblée comprenant un train d'impulsions de durée D₄.

Selon ce mode de réalisation, l'amplitude des impulsions A₄ est comprise dans la gamme de valeurs [2 MPa ; 8MPa] pendant une durée D₄ comprise dans la gamme de valeurs [1ms - 50ms] de sorte à stimuler une zone ciblée Z_{C} en limitant les dommages mécaniques et thermiques. La génération d'une activité électrique pouvant induire une repolarisation d'un ventricule ou d'une oreillette est ainsi liée à la poussée ultrasonore appliquée sur les tissus qui se contractent.

La figure 6B représente un second faisceau focalisé 51 appliqué dans une zone ciblée comprenant un train d'impulsions de durée D_{4'}.

Selon ce mode de réalisation, l'amplitude des impulsions A₄' est comprise dans la gamme de valeurs [8 MPa ; 12MPa] pendant une durée D₄' comprise dans la gamme de valeurs [50µs - 1ms] de sorte à stimuler une zone ciblée Z_{C} en limitant les dommages thermiques et thermiques. Dans ce cas, la génération d'une activité électrique pouvant induire une repolarisation d'un ventricule ou d'une oreillette est ainsi liée au phénomène de cavitation dont les microbulles permettent aux tissus de se contracter.

Selon des tests réalisés, les configurations suivantes ont permis d'aboutir à des stimulations de tissus du coeur dans 90% des cas. Dans ces tests, chaque stimulation n'a causé aucun dommage thermique ni mécanique dans la zone de focalisation et/ou à sa proximité :
▪ Une première configuration permettant de générer un niveau de pression dans la zone focalisée dont l'amplitude positive du signal acoustique est de 6MPa, l'amplitude négative du signal acoustique correspondante étant de 4MPa, pendant une durée d'impulsion de 1ms. Les valeurs numériques des pressions acoustiques et de la durée de l'impulsion pouvant varier de 10% de leur valeur nominale dans cet exemple ;
▪ Une seconde configuration permettant de générer un niveau de pression dans la zone focalisée dont l'amplitude positive du signal acoustique est de 10MPa, l'amplitude négative du signal acoustique correspondante étant de 6MPa, pendant une durée d'impulsion allant de 50µ à 1ms. Les valeurs numériques de la pression acoustique peuvent varier de 10% de leurs valeurs nominales dans cet exemple.

Lorsqu'un agent de contraste ultrasonore est utilisé, une diminution des seuils de pression acoustique peut être obtenue par une diminution de la puissance des signaux focalisés dans la zone focalisée. Une diminution de quelques centièmes de MPa à quelques MPa peut être obtenue en fonction :
▪ du mode d'administration de l'agent de contraste ;
▪ de sa quantité ;
▪ des données physiologiques du patient ou encore ;
▪ de la période à laquelle le faisceau est générée après l'administration de l'agent de contraste.

### Applications à la stimulation cardiaque

Le procédé selon l'invention peut être utilisé selon diverses applications nécessitant une stimulation cardiaque.

En outre, le procédé de stimulation cardiaque peut être appliqué à d'autres fins que des applications thérapeutiques. A titre d'exemple, le procédé de stimulation cardiaque peut être appliqué pour cartographier une région du coeur électriquement en analysant des déformations tissulaires sur cette région. Une autre application concerne l'analyse du phénomène de cavitation dans des régions du coeur. D'autres applications sont possibles.

Une première application concerne la resynchronisation cardiaque. Chaque impulsion du faisceau de signaux ultrasonore est alors synchronisée sur le rythme de l'ECG, chaque impulsion étant générée entre la fin de la repolarisation et le début de la dépolarisation d'un ventricule ou d'une oreillette.

Lorsque l'on cherche à resynchroniser les ventricules droit et gauche, un premier mode de réalisation comprend le paramétrage d'au moins un faisceau focalisé dans une zone ciblée d'un ventricule lors de la repolarisation du ventricule concerné et prenant en compte le décalage de désynchronisation des deux ventricules. Ainsi la stimulation peut générer une activité électrique créant ainsi un nouveau complexe QRS synchronisé entre les deux ventricules.

Un second mode de réalisation comprend le paramétrage du réseau de phase pour au moins créer un faisceau focalisé dans chacun des ventricules. Dans ce cas les deux faisceaux sont générés lors de la repolarisation des deux ventricules mais sont espacés d'une durée compensant la désynchronisation des ventricules.

Le même procédé de resynchronisation des oreillettes peut être appliqué en générant un ou des faisceaux focalisés pendant les périodes de repolarisation des oreillettes.

La stimulation du coeur provoquant la resynchronisation des ventricules ou des oreillettes peut être générée par une ou plusieurs impulsions telles que définies dans l'un des deux cas de figures décrits aux figures 4 à 6B.

Le procédé de stimulation de l'invention permet de simuler un placement d'électrodes ou de sondes fixées au coeur générant des impulsions pour assurer une resynchronisation permanente des ventricules ou des oreillettes. Cette application est particulièrement avantageuse pour la détection de zones du coeur susceptibles de recevoir des sondes implantées qui seront fixées à la surface du coeur. Les sondes implantées servent notamment pour la détection d'une désynchronisation ventriculaire ou auriculaire pour générer des impulsions à partir d'un composant également implanté sous la peau. Un problème identifié des dispositifs implantés actuels est que les sondes sont parfois fixées sur des zones non répondantes.

Le procédé de stimulation de l'invention peut donc permettre de choisir des zones réactives à la stimulation avant leur pose définitive.

Une application du procédé de l'invention permet de contrôle le bon fonctionnement du coeur par exemple après une resynchronisation cardiaque. A cet effet, le système d'imagerie, par exemple l'IRM, permet de mesurer l'amélioration de la fonction cardiaque en mesurant par exemple la fraction de sang éjectée par le coeur.

Une seconde application du procédé de stimulation concerne la génération d'une fibrillation auriculaire ou ventriculaire. Chaque impulsion du faisceau focalisé est synchronisée sur une période pendant laquelle les tissus cardiaques se polarisent en particulier au moment de la génération de l'onde T. La stimulation provoquant la fibrillation peut être générée par une ou plusieurs impulsions telles que définies dans l'un des deux cas de figures décrits aux figures 4 à 6B.

Une troisième application concerne la détection d'une ou plusieurs zones particulières du coeur générant une arythmie cardiaque. Lorsqu'une arythmie est formée, il est possible de repérer sur l'ECG l'instant auquel elle apparaît, cet instant est noté T_{F}. Chaque impulsion est alors synchronisée sur l'instant identifié T_{F} déterminé sur l'ECG. Le procédé de stimulation est alors réalisé en différentes zones du coeur. Par analyse de la réponse électrique survenant conséquemment à la génération d'un faisceau focalisé, il est possible de déterminer si la zone ciblée correspond à la zone générant l'extrasystole. De proche en proche, par application de différentes stimulations dans différentes zones ciblées, il est possible d'identifier la zone génératrice de l'extrasystole. L'analyse de l'ECG peut révéler un dérèglement électriquement permettant de vérifier que la zone stimulée cause une arythmie. Les stimulations permettant la détection d'une zone génératrice d'une extrasystole peuvent être générées par une ou plusieurs impulsions telles que définies dans l'un des deux cas de figures décrits aux figures 4 à 6B de manière à contrôler et prévenir les dommages thermiques et mécaniques des tissus.

Une quatrième application concerne la détection d'une ou de plusieurs zones non répondante(s) électriquement du coeur. Par exemple, le procédé de stimulation de l'invention peut être appliqué après une ablation d'une zone du coeur pour vérifier que la partie ablatée, brûlée ou nécrosée est une zone non répondante ou pour vérifier qu'une zone non répondante n'est justement plus présente. Une analyse de la réponse électrique, par exemple, au moyen d'un cathéter électrique permet de déduire localement l'activité électrique. Lorsque l'impulsion est d'une durée supérieure à 1ms, correspondant aux impulsions du premier cas de figure, une table de correspondance entre la déformation des tissus et l'activité électrique peut être utilisée. Dans ce dernier cas un cathéter électrique n'est pas nécessaire sauf à l'utiliser en complément d'une imagerie permettant de visualiser les déplacements des tissus, telle que l'IRM.

Une cinquième application concerne celle de la modification de la fréquence de battement du coeur à partir d'au moins une stimulation cardiaque générée par le procédé de l'invention. Cette application est appelée également dans la terminologie anglo-saxonne médicale « pacing ».

Dans ce cas, le procédé peut être utilisé pour augmenter la fréquence du rythme cardiaque. Chaque impulsion du faisceau de signaux ultrasonores est alors émise à une fréquence différente de la fréquence déterminée sur l'ECG de manière à synchroniser le rythme cardiaque sur les impulsions du faisceau focalisé. L'un des deux cas de figures exposés aux figures 4 à 6B peut être utilisé notamment par des trains d'impulsions successifs sur des périodes de temps données. Cette application peut se substituer à un massage cardiaque.

### Système - récapitulatif

L'invention concerne un système tel que celui décrit dans la figure 1 permettant de mettre en oeuvre le procédé de contrôle pour la calibration de faisceau focalisé ou le procédé de stimulation pour une des applications précitées. Enfin, notons que le système de l'invention est modulaire et peut être utilisé de différentes manières pour résoudre le problème de la stimulation contrôlée de l'invention.

Selon une première configuration, un système d'imagerie IMG utilisant une IRM permet de générer une image en coupe 2D ou 3D du coeur. La position de la zone ciblée peut permettre de n'asservir la génération que d'une portion de l'image, notamment celle qui concerne l'application du faisceau focalisé. Ainsi, il est possible de contrôler visuellement la zone focalisée sur une tranche d'image comprenant cette zone.

Selon un cas, le système d'imagerie IRM est configuré pour déterminer localement dans ou à proximité de la zone ciblée les variations de températures, mode appelé MR-T. Il peut être également configuré pour déterminer localement dans ou à proximité de la zone ciblée les variations de déplacements tissulaires, mode appelé MR-ARFI. Les deux modes MR-T et MR-ARFI sont compatibles pour être configurés dans une même application.

Selon un autre cas, le contrôle de température est effectué au moyen d'un système d'imagerie par IRM comme précédemment et le contrôle des déformations tissulaires est par exemple réalisé au moyen d'un cathéter introduit dans le coeur et mesurant la pression locale. Les déformations tissulaires peuvent également être déduites d'un système d'imagerie à ultrasons.

Un système de positionnement peut être conçu à partir de la définition d'un point sur l'image et par détermination des coordonnées dans un référentiel du coeur d'une position ciblée. Cette position de la zone ciblée est ensuite transmise au réseau de phases RES_US pour la génération d'un faisceau focalisé qui est ainsi asservi dynamiquement et automatiquement sur cette position.

Le système comprend, selon un mode de réalisation, un dispositif de mesure d'un niveau de cavitation SYS_CAV, par exemple au moyen d'un dispositif ultrasonore comme détaillé précédemment.

Selon une configuration l'ECG est acquis au moyen d'électrodes positionnées, par exemple, sur un patient. L'ECG peut également être acquis par tout autre moyen connu permettant de restituer la fréquence cardiaque.

Un même système d'acquisition de l'activité électrique du coeur peut être utilisé pour déterminer l'ECG et les réponses électriques dues aux stimulations générées par le procédé de l'invention.

### Dispositif portable de stimulation cardiaque

Enfin un mode particulier concerne un dispositif portable comprenant des configurations de signaux prédéterminés tels que ceux décrits dans les deux cas de figures exposés aux figures 4 à 6B. Ce dispositif est illustré à la figure 7. Il comprend un générateur de signaux ultrasonores RES_US qui peut générer un unique signal ou une pluralité de signaux formant un faisceau focalisé. On note que dans le cas où le faisceau ne comprend qu'un seul signal, il est focalisé. Le faisceau est préconfiguré de sorte à se focaliser à une certaine distance de l'émetteur. On note le générateur RES_US comme sur la figure 1 bien que dans ce mode de réalisation, le générateur de signaux ultrasonores n'est pas nécessairement un réseau de phase. Selon un mode de réalisation, une pluralité de distances focales peuvent définir différentes configurations, par exemple différentes distances comprises entre 2 et 20cm.

Le dispositif comprend des moyens électriques SYS_ELEC_1 pour déterminer la fréquence de battement du coeur à minima. Selon un mode amélioré, les moyens électriques SYS_ELEC_1 permettent de déterminer un ECG complet.

Le dispositif est conçu pour être apposé à la surface de la peau. Selon le gabarit du patient, une distance focale est déterminée par un opérateur. Le dispositif comprend un moyen d'activation du faisceau selon un cas de figure donné, la génération du faisceau est synchronisée sur la fréquence cardiaque et sur l'ECG lorsqu'il déterminé. Lorsqu'uniquement la fréquence cardiaque est utilisée, le moment de repolarisation des tissus cardiaques est calculé à partir d'un calculateur du dispositif permettant d'évaluer la fenêtre temporelle correspondant à la repolarisation des ventricules et/ou des oreillettes. Lorsqu'aucune fréquence cardiaque n'est disponible, ce qui correspond à un arrêt cardiaque, le dispositif ne se synchronise pas sur la fréquence cardiaque ou sur l'ECG et permet la génération d'un faisceau focalisé.

Selon un mode de réalisation, différentes pré-configurations de faisceaux sont présentes dans le dispositif.

Dans un exemple de réalisation, il existe un premier mode comprenant 3 configurations de faisceau correspondant au premier cas de figure (impulsions dites longues, amplitudes dites faibles correspondant au cas de la figure 6A) :
Une première configuration comprend l'émission d'une impulsion, une seconde configuration comprend l'émission de trois impulsions, une troisième configuration comprend l'émission de 6 impulsions.

Dans un second mode comprenant 3 configurations de faisceau correspondant au second cas de figure (impulsions dites courtes, amplitudes dites fortes correspondant au cas de la figure 6B) :
Une première configuration comprend l'émission d'une impulsion, une seconde configuration comprend l'émission de trois impulsions, une troisième configuration comprend l'émission de 6 impulsions.

Le dispositif de l'invention comprend en outre un capteur permettant de détecter qu'une contraction mécanique du coeur est présente. A titre d'exemple un capteur de pression sanguine permet de détecter qu'une contraction du coeur a lieu. Ce capteur peut être positionné sur le poignet pour mesurer le pouls ou sur le torse pour détecter la présence d'un battement.

Ainsi, l'invention concerne un dispositif portable préconfiguré qui peut être utilisé dans des situations d'urgence. Il peut par exemple, être embarqué dans un camion de pompier ou être disposé dans des lieux dans lesquels un accident cardiaque peut survenir chez une personne.

Ce dispositif embarqué ne comprend pas nécessairement de système d'imagerie lorsqu'on veut limiter son encombrement. Le système d'imagerie peut être utilisé lors de la pré-configuration des signaux lors de la mise en production du dispositif.

## Revendications

1. Système de stimulation cardiaque ultrasonore comprenant :
▪ un moyen de mesure de l'activité électrique du coeur pour l'acquisition d'un électrocardiogramme (ECG) ;
- un moyen de génération d'un faisceau de signaux ultrasonores focalisé (Fus) sur une zone ciblée (Z_{c}), lesdits signaux étant calibrés pour générer une stimulation électrique dans une zone du coeur, ladite génération du faisceau étant synchrone avec un premier instant choisi de l'électrocardiogramme (ECG), la génération du faisceau correspondant à au moins une impulsion générée dans une zone focalisée (ZF), une amplitude des impulsions étant configurée de sorte que la pression acoustique appliquée dans la zone focalisée (ZF) est comprise :
o dans une première gamme de pressions (GP2) de [2 - 8 MPa] pour des durées d'impulsions comprises dans une première gamme de durées (GD2) [1ms - 50ms], ou
▪ dans une seconde gamme de pressions (GP3) de [6 - 12 MPa] pour des durées d'impulsions comprises dans une seconde gamme de durées (GD3) [50µs - 1ms] et pour une durée d'application supérieure à 50 µs et inférieure à une durée seuil d'impulsion calculée pour une amplitude donnée de l'impulsion;
▪ un moyen de localisation de la zone ciblée (Z_{c}) couplé à un moyen de positionnement du moyen de génération du faisceau focalisé (Fus) de sorte à asservir ledit faisceau de signaux ultrasonores focalisé (Fus) dans la zone ciblée (Z_{c}), ledit moyen de localisation étant synchronisé avec le moyen de génération du faisceau de signaux focalisés (Fus);
▪ un même moyen de contrôle capable de suivre en temps réel une température et une déformation tissulaire dans la zone ciblée (Z_{c}), ledit moyen de contrôle relevant des mesures de manière synchrone avec le rythme de l'électrocardiogramme.

2. Système selon la revendication 1, dans lequel la génération du faisceau de signaux ultrasonores est engagée de sorte que la au moins une impulsion soit générée lors de la repolarisation des tissus cardiaques dans la zone ciblée.

3. Système selon la revendication 1, dans lequel la génération du faisceau de signaux ultrasonores est engagée de sorte que la au moins une impulsion soit générée lors de l'onde T de l'ECG.

4. Système selon l'une des revendications 1 à 3, dans lequel la température dans la zone focalisée (Z_{F}) et la déformation tissulaire sont déterminées par un même système d'imagerie, ledit système d'imagerie étant un système d'imagerie IRM, les données acquises par le système d'imagerie IRM permettant de déduire une déformation locale du tissu induite par la pression ultrasonore générée par le faisceau de signaux ultrasons et une élévation de température locale induite par l'énergie générée localement par le faisceau de signaux ultrasons.

5. Système selon l'une des revendications 1 à 4, **caractérisé en ce que** le système de positionnement et le moyen de contrôle de la température sont un même système d'imagerie IRM.

6. Système selon l'une des revendications 1 à 5, **caractérisée en ce qu'**un second contrôle de zones voisines (Zv) à la zone focalisée (Z_{F}) est réalisé, ledit second contrôle comportant la mesure d'au moins un des paramètres suivants en temps réel :
▪ une température d'au moins une zone voisine (Zv) de la zone focalisée (Z_{F}) et ;
▪ une déformation tissulaire dans au moins une zone voisine (Zv) de la zone focalisée (Z_{F}) en réponse à au moins une impulsion du premier faisceau focalisé (Fus) et/ou ;

7. Système selon l'un des revendications 1 à 6, dans lequel ledit système comprend un dispositif ultrasonore (SYS_CAV) pour la détection d'un niveau de cavitation (ACQ_CAV) dans ou à proximité de la zone focalisée, ledit dispositif ultrasonore étant un capteur d'ondes ultrasonores mesurant les ondes émises par le moyen de génération d'un faisceau de signaux ultrasonores.

8. Système selon la revendication 7, dans lequel ledit système comprend un système électrique pour la mesure de l'activité électrique (ACQ_AE) dans et/ou à proximité de la zone focalisée, le calculateur étant configuré pour centraliser et suivre, pendant ou après la génération du faisceau ultrasonore, l'activité électrique dans et/ou à proximité de la zone focalisée en réponse à la génération du faisceau de signaux ultrasonores.

9. Système selon l'une des revendications 7 à 8, dans lequel le calculateur est configuré pour déterminer, pendant ou après la génération du faisceau de signaux ultrasonores, si la zone ciblée est électriquement sensible, ladite détermination étant réalisée sur la base de la déformation tissulaire mesurée en réponse à la génération du faisceau de signaux ultrasonores, ou sur la base du niveau de cavitation mesuré en réponse à la génération du faisceau de signaux ultrasonores.

10. Système selon la revendication 9, dans lequel la détermination de la sensibilité électrique de la zone ciblée comprend une comparaison de la déformation tissulaire mesurée ou de l'activité électrique ou du niveau de cavitation mesuré par rapport à un seuil prédéterminé pour une amplitude et une durée données des impulsions, ladite zone du coeur étant **caractérisée** comme électriquement « non-répondante » lorsque la déformation tissulaire mesurée ou l'activité électrique mesurée ou le niveau de cavitation mesuré dans la zone ciblée est inférieur(e) au seuil prédéterminé.

11. Système selon l'une des revendications 8 à 10, dans lequel le calculateur est configuré pour déterminer, pendant ou après la génération du faisceau de signaux ultrasonores, un indicateur d'activité mécano-électrique du tissu cardiaque de la zone ciblée, ledit indicateur étant déterminé sur la base d'une corrélation de l'activité électrique générée par le faisceau focalisé (FUS) et mesurée dans ou à proximité de la zone ciblée (Zc) avec les mesures de déformations tissulaires dans la même zone ciblée (Zc) obtenues par le système d'imagerie.

12. Système selon l'une des revendication 8 à 11, dans lequel le moyen de génération d'un faisceau de signaux ultrasonores focalisés est un réseau de phase couplé au moyen de mesure de l'activité électrique, l'asservissement dudit faisceau de signaux ultrasonores focalisé (FUS) au moyen du système de positionnement permettant de mesurer, pendant la génération du faisceau de signaux ultrasonores, des déplacements de la zone ciblée du coeur dans un référentiel lié au réseau de phase en réponse à la génération du faisceau de signaux ultrasonores.

13. Système selon la revendication 12, dans lequel le calculateur est configuré pour calibrer, pendant ou après la génération du faisceau de signaux ultrasonores, un premier signal focalisé (FUS) comprenant une pluralité d'impulsions, chaque impulsion ayant une première durée (D1) et une première amplitude (A), ledit signal étant appliqué pendant une durée (D2) dans une zone ciblée (Zc), la première amplitude (A) et la première durée (D1) étant définies en fonction d'au moins une donnée parmi lesquelles :
▪ une consigne de température dans la zone focalisée (Z_{F}) et/ou sur les zones voisines et/ou sur les côtes de la paroi transcostale ; et/ou
▪ une consigne de la déformation tissulaire dans la zone focalisée (Z_{F}) et/ou ;
▪ une consigne d'un niveau de cavitation dans la zone focalisée (ZF) et/ou ;
▪ une détection d'au moins un déplacement lié au mouvement de la zone focalisée dans un repère lié au réseau de phase et/ou ;
▪ une consigne d'activité électrique dans la zone focalisée (Z_{F}).

14. Système selon la revendication 13, dans lequel le faisceau de signaux focalisés est généré dans différentes zones ciblées (Zc) du coeur, le calculateur étant configuré pour réaliser :
▪ Un relevé de différentes valeurs représentant soit des déformations tissulaires de chaque zone ciblée, soit des niveaux électriques mesurés à proximité ou dans chaque zone ciblée
▪ Un relevé des temps de réponse électrique ou de déformations de chaque zone ciblée ;
▪ Une calibration pour chaque zone ciblée d'un signal selon la revendication 13, lesdits signaux étant configurés entre eux avec un délai temporel dépendant des relevés des temps de réponse.

15. Système selon la revendication 8 à 14, dans lequel le calculateur est configuré pour, pendant la génération du faisceau de signaux ultrasonores :
▪ comparer le niveau de cavitation mesuré à un premier seuil et la déformation tissulaire mesurée à un second seuil, et
▪ couper automatiquement la génération du signal ultrasonore lorsque le premier et le deuxième seuil sont dépassés.

## Patentansprüche

1. Ultraschall-Herzschrittmachersystem, bestehend aus:
- einem Mittel zur Messung der elektrischen Aktivität des Herzens zur Erfassung eines Elektrokardiogramms (EKG);
- einem Mittel zur Erzeugung eines auf einen Zielbereich (Zc) fokussierten Ultraschallsignalstrahls (Fus), wobei die Signale kalibriert sind, um eine elektrische Stimulation in einem Bereich des Herzens zu erzeugen, wobei die Strahlerzeugung synchron mit einem ersten ausgewählten Zeitpunkt des Elektrokardiogramms (EKG) ist, wobei die Strahlerzeugung mindestens einem Impuls entspricht, der in einem fokussierten Bereich (ZF) erzeugt wird, wobei eine Amplitude der Impulse so konfiguriert ist, dass der in dem fokussierten Bereich (ZF) ausgeübte Schalldruck zwischen folgenden Druckbereichen liegt:
∘ in einem ersten Druckbereich (GP2) von [2 - 8 MPa] für Impulsdauern, die in einem ersten Zeitraum (GD2) [1ms-50ms] liegen, oder
∘ in einem zweiten Druckbereich (GP3) von [6 - 12 MPa] für Impulsdauern, die in einem zweiten Zeitraum (GD3) [50 µs - 1ms] liegen, und für eine Anwendungsdauer, die länger als 50 µs und kürzer als eine Impulsschwellendauer ist, die für eine gegebene Amplitude des Impulses berechnet wird;
- einem Mittel zur Lokalisierung des Zielbereichs (Z_{c}), das mit einem Mittel zur Positionierung des Mittels zur Erzeugung des fokussierten Strahls (Fus) gekoppelt ist, um den fokussierten Ultraschallsignalstrahl (Fus) in den Zielbereich (Zc) zu steuern, wobei das Mittel zur Lokalisierung mit dem Mittel zur Erzeugung des fokussierten Signalstrahls (Fus) synchronisiert ist;
- einem gleichen Überwachungsmittel, das in der Lage ist, eine Temperatur und eine Gewebeverformung in dem Zielbereich (Z_{c}) in Echtzeit zu verfolgen, wobei das Überwachungsmittel Messungen synchron mit dem Rhythmus des Elektrokardiogramms erhebt.

2. System nach Anspruch 1, bei dem die Erzeugung des Ultraschallsignalstrahls so eingeleitet wird, dass der mindestens eine Impuls während der Repolarisation des Herzgewebes im Zielbereich erzeugt wird.

3. System nach Anspruch 1, bei dem die Erzeugung des Ultraschallsignalstrahls so eingeleitet wird, dass der mindestens eine Impuls während der T-Welle des EKGs erzeugt wird.

4. System nach einem der Ansprüche 1 bis 3, bei dem die Temperatur in der fokussierten Zone (ZF) und die Gewebeverformung durch ein und dasselbe Bildgebungssystem bestimmt werden, wobei das Bildgebungssystem ein MRT-Bildgebungssystem ist, wobei aus den vom MRT-Bildgebungssystem erfassten Daten eine lokale Gewebeverformung, die durch den vom Ultraschallsignalstrahl erzeugten Ultraschalldruck induziert wird, und ein lokaler Temperaturanstieg, der durch die lokal vom Ultraschallsignalstrahl erzeugte Energie induziert wird, abgeleitet werden können.

5. System nach einem der Ansprüche 1 bis 4, **dadurch gekennzeichnet, dass** das Positionierungssystem und das Mittel zur Temperaturregelung ein und dasselbe MRT-Bildgebungssystem sind.

6. System nach einem der Ansprüche 1 bis 5, **dadurch gekennzeichnet, dass** eine zweite Kontrolle von an die fokussierte Zone (Z_{F}) angrenzenden Zonen (Zv) durchgeführt wird, wobei die zweite Kontrolle die Messung von mindestens einem der folgenden Parameter in Echtzeit umfasst:
- eine Temperatur von mindestens einer benachbarten Zone (Zv) der fokussierten Zone (Z_{F}) und;
- eine Gewebeverformung in mindestens einem benachbarten Bereich (Zv) des fokussierten Bereichs (Z_{F}) als Reaktion auf mindestens einen Impuls des ersten fokussierten Strahls (Fus) und/oder;

7. System nach einem der Ansprüche 1 bis 6, bei dem das System eine Ultraschallvorrichtung (SYS_CAV) zum Erfassen eines Kavitationspegels (ACQ_CAV) in oder in der Nähe der fokussierten Zone umfasst, wobei die Ultraschallvorrichtung ein Ultraschallwellensensor ist, der die von den Mitteln zum Erzeugen eines Ultraschallsignalstrahls ausgesendeten Wellen misst.

8. System nach Anspruch 7, bei dem das System ein elektrisches System zur Messung der elektrischen Aktivität (ACQ_AE) in und/oder in der Nähe der fokussierten Zone umfasst, wobei der Rechner so konfiguriert ist, dass er während oder nach der Erzeugung des Ultraschallstrahls die elektrische Aktivität in und/oder in der Nähe der fokussierten Zone als Reaktion auf die Erzeugung des Ultraschallsignalstrahls zentralisiert und verfolgt.

9. System nach einem der Ansprüche 7 bis 8, bei dem der Rechner so konfiguriert ist, dass er während oder nach der Erzeugung des Ultraschallsignalstrahls bestimmt, ob der Zielbereich elektrisch empfindlich ist, wobei die Bestimmung auf der Grundlage der als Reaktion auf die Erzeugung des Ultraschallsignalstrahls gemessenen Gewebeverformung oder auf der Grundlage des als Reaktion auf die Erzeugung des Ultraschallsignalstrahls gemessenen Kavitationspegels durchgeführt wird.

10. System nach Anspruch 9, bei dem die Bestimmung der elektrischen Empfindlichkeit des Zielbereichs einen Vergleich der gemessenen Gewebeverformung oder der gemessenen elektrischen Aktivität oder des gemessenen Kavitationspegels mit einem vorbestimmten Schwellenwert für eine gegebene Amplitude und Dauer der Impulse umfasst, wobei der Herzbereich als elektrisch "nicht ansprechend" gekennzeichnet ist, wenn die gemessene Gewebeverformung oder die gemessene elektrische Aktivität oder der gemessene Kavitationspegel im Zielbereich unter dem vorbestimmten Schwellenwert liegt.

11. System nach einem der Ansprüche 8 bis 10, bei dem der Rechner so konfiguriert ist, dass er während oder nach der Erzeugung des Ultraschallsignalstrahls einen Indikator für die mechanoelektrische Aktivität des Herzgewebes im Zielbereich bestimmt, wobei der Indikator auf der Grundlage einer Korrelation der durch den fokussierten Strahl (FUS) erzeugten und in oder nahe dem Zielbereich (Zc) gemessenen elektrischen Aktivität mit den durch das Bildgebungssystem erhaltenen Messungen der Gewebeverformungen im selben Zielbereich (Zc) bestimmt wird.

12. System nach einem der Ansprüche 8 bis 11, bei dem das Mittel zur Erzeugung eines fokussierten Ultraschallsignalstrahls ein Phasenarray ist, das mit dem Mittel zur Messung der elektrischen Aktivität gekoppelt ist, wobei die Steuerung des fokussierten Ultraschallsignalstrahls (FUS) mittels des Positionierungssystems während der Erzeugung des Ultraschallsignalstrahls die Messung von Verschiebungen des Zielbereichs des Herzens in einem mit dem Phasenarray verbundenen Bezugssystem als Reaktion auf die Erzeugung des Ultraschallsignalstrahls ermöglicht.

13. System nach Anspruch 12, bei dem der Rechner so konfiguriert ist, dass er während oder nach der Erzeugung des Ultraschallsignalstrahls ein erstes fokussiertes Signal (FUS) kalibriert, das eine Vielzahl von Impulsen umfasst, wobei jeder Impuls eine erste Dauer (01) und eine erste Amplitude (A) hat, wobei das Signal für eine Dauer (02) in einem Zielbereich (Zc) angelegt wird, wobei die erste Amplitude (A) und die erste Dauer (01) in Abhängigkeit von mindestens einem der folgenden Daten definiert werden:
- einen Temperatursollwert in der fokussierten Zone (ZF) und/oder an den benachbarten Zonen und/oder an den Rippen der transkostalen Wand; und/oder
- einen Sollwert für die Gewebeverformung in der fokussierten Zone (ZF) und/oder;
- eine Vorgabe eines Kavitationspegels in der fokussierten Zone (ZF) und/oder;
- eine Erfassung von mindestens einer mit der Bewegung der fokussierten Zone verbundenen Verschiebung in einem mit dem Phasenarray verbundenen Koordinatensystem und/oder ;
- ein Sollwert für die elektrische Aktivität in der fokussierten Zone (ZF).

14. System nach Anspruch 13, bei dem der fokussierte Signalstrahl in verschiedenen Zielbereichen (Zc) des Herzens erzeugt wird, wobei der Rechner so konfiguriert ist, dass er Folgendes ausführt:
- Eine Aufzeichnung verschiedener Werte, die entweder Gewebeverformungen in jedem Zielbereich oder elektrische Pegel darstellen, die in der Nähe oder in jedem Zielbereich gemessen wurden
- Eine Aufzeichnung der elektrischen Reaktionszeiten oder der Verformungen jedes Zielbereichs;
- Eine Kalibrierung für jeden Zielbereich eines Signals nach Anspruch 13, wobei die Signale untereinander mit einer zeitlichen Verzögerung konfiguriert sind, die von den Aufzeichnungen der Reaktionszeiten abhängt.

15. System nach Anspruch 8 bis 14, wobei der Rechner so konfiguriert ist, dass er während der Erzeugung des Ultraschallsignalstrahls:
- den an einem ersten Schwellenwert gemessenen Kavitationspegel und die an einem zweiten Schwellenwert gemessene Gewebeverformung vergleicht, und
- die Erzeugung des Ultraschallsignals automatisch abschaltet, wenn der erste und der zweite Schwellenwert überschritten werden.

## Claims

1. An ultrasonic cardiac stimulation system comprising:
- a means for measuring electrical activity of the heart in order to acquire an electrocardiogram (ECG);
- a means for generating a beam of ultrasonic signals (Fus) focused on a targeted zone (Zc), said signals being calibrated to generate electrical stimulation in a zone of the heart, said generating the beam being synchronous with a first selected instant of the electrocardiogram (ECG), generating the beam corresponding to at least one pulse generated in a focused zone (Z_{F}), an amplitude of the pulses being configured so that the acoustic pressure applied in the focused zone (Z_{F}) is included:
∘ in a first pressure range (GP2) of [2 - 8 MPa] for pulse durations within a first duration range (GD2) [1 ms - 50 ms], or
∘ in a second pressure range (GP3) of [6 - 12 MPa] for pulse durations within a second duration range (GD3) [50µs - 1ms] and for an application duration greater than 50 µs and less than a pulse threshold duration calculated for a given pulse amplitude;
- a means for locating the targeted zone (Zc) coupled to means for positioning the means for generating the focused beam (Fus) so as to feedback control said focused beam of ultrasound signals (Fus) in the targeted zone (Zc), said locating means being synchronised with the means for generating the focused signal beam (Fus);
- a same monitoring means capable of following in real time temperature and tissue deformation in the targeted zone (Zc), said monitoring means reading measurements synchronously with the rhythm of the electrocardiogram.

2. A system according to claim 1, wherein generating the beam of ultrasound signals is engaged such that the at least one pulse is generated upon repolarisation of cardiac tissues in the targeted zone.

3. The system according to claim 1, wherein generating the beam of ultrasound signals is engaged such that the at least one pulse is generated during the ECG T-wave.

4. The system according to one of claims 1 to 3, wherein the temperature in the focused zone (ZF) and the tissue deformation are determined by a same imaging system, said imaging system being an MRI imaging system, the data acquired by the MRI imaging system making it possible to deduce local deformation of the tissue induced by the ultrasound pressure generated by the beam of ultrasound signals and a local temperature rise induced by energy locally generated by the beam of ultrasound signals.

5. The system according to one of claims 1 to 4, **characterised in that** the positioning system and the temperature monitoring means are one and the same MRI imaging system.

6. The system according to one of claims 1 to 5, **characterised in that** a second monitoring of zones (Zᵥ) next to the focused zone (Z_{F}) is carried out, said second monitoring including measuring at least one of the following parameters in real time:
- a temperature of at least one zone (Zᵥ) next to the focused zone (Z_{F}) and;
- a tissue deformation in at least one zone (Zᵥ) next to the focused zone (Z_{F}) in response to at least one pulse of the first focused beam (Fus) and/or;

7. The system according to one of claims 1 to 6, wherein said system comprises an ultrasonic device (SYS_CAV) for the detection of a cavitation level (ACQ_CAV) in or in proximity to the focused zone, said ultrasonic device being an ultrasonic wave sensor measuring waves emitted by the means for generating a beam of ultrasonic signals.

8. The system according to claim 7, wherein said system comprises an electrical system for the measurement of electrical activity (ACQ _AE) in and/or in proximity to the focused zone, the calculator being configured to centralise and follow, during or after generating the ultrasonic beam, electrical activity in and/or in proximity to the focused zone in response to generating the beam of ultrasonic signals.

9. The system according to one of claims 7 to 8, wherein the calculator is configured to determine, during or after generation of the beam of ultrasound signals, whether the targeted zone is electrically sensitive, said determination being made on the basis of the tissue deformation measured in response to generating the beam of ultrasound signals, or on the basis of the cavitation level measured in response to generating the beam of ultrasound signals.

10. The system according to claim 9, wherein determining the electrical sensitivity of the targeted zone comprises comparing the measured tissue deformation or electrical activity or cavitation level against a predetermined threshold for a given pulse amplitude and duration, said zone of the heart being characterised as electrically "non-responsive" when the measured tissue deformation or electrical activity or cavitation level in the targeted zone is less than the predetermined threshold.

11. The system according to one of claims 8 to 10, wherein the calculator is configured to determine, during or after generation of the beam of ultrasound signals, an indicator of mechano-electrical activity of the cardiac tissue of the targeted zone, said indicator being determined on the basis of a correlation of the electrical activity generated by the focused beam (FUS) and measured in or in proximity to the targeted zone (Zc) with the measurements of tissue deformations in the same targeted zone (Zc) obtained by the imaging system.

12. The system according to one of claims 8 to 11, wherein the means for generating a beam of focused ultrasound signals is a phase grating coupled to the means for measuring electrical activity, feedback control of said focused beam of ultrasound signals (FUS) by means of the positioning system making it possible to measure, during generation of the beam of ultrasound signals, displacements of the targeted zone of the heart in a reference frame related to the phase grating in response to generating the beam of ultrasound signals.

13. The system according to claim 12, wherein the calculator is configured to calibrate, during or after generation of the beam of ultrasonic signals, a first focused signal (FUS) comprising a plurality of pulses, each pulse having a first duration (D1) and a first amplitude (A), said signal being applied for a duration (D2) in a targeted zone (Zc), the first amplitude (A) and the first duration (D1) being defined as a function of at least one piece of data among which:
- a temperature setpoint in the focused zone (Z_{F}) and/or on the neighbouring zones and/or on the ribs of the transcostal wall; and/or
- a tissue deformation setpoint in the focused zone (Z_{F}) and/or;
- a cavitation level setpoint in the focused zone (Z_{F}) and/or;
- detection of at least one displacement related to the movement of the focused zone in a reference frame related to the phase grating and/or;
- an electrical activity setpoint in the focused zone (Z_{F}).

14. The system according to claim 13, wherein the beam of focused signals is generated in different targeted zones (Zc) of the heart, the calculator being configured to perform:
- reading of different values representing either tissue deformations of each targeted zone, or electrical levels measured in proximity to or in each targeted zone
- reading of the electrical response times or deformations in each targeted zone;
- calibration for each targeted zone of a signal according to claim 13, said signals being configured with a time delay depending on the response time readings.

15. The system according to claims 8 to 14, wherein the calculator is configured to, during generation of the beam of ultrasound signals:
- compare the cavitation level measured at a first threshold with the tissue deformation measured at a second threshold, and
- automatically cut off generation of the ultrasound signal when the first and second thresholds are exceeded.
